# EUROPEAN PATENT APPLICATION

(11) **EP 4 736 848 A1**
(43) Date of publication of application: **06.05.2026**
(21) Application number: 24830981.7
(22) Date of filing: 28.06.2024
(51) Int. Cl.: A61K 9/51, C07C 229/12, C07C 229/16, A61K 47/14, A61K 47/28, A61K 9/127

(54) **PHARMACEUTICAL COMPOSITION CONTAINING CATIONIC LIPID AND USE THEREOF**

(30) Priority: 28.06.2023 CN 202310780219
(71) Applicant: Shanghai Regenelead Therapies Co., Ltd., Shanghai 201206 (CN)
(72) Inventor: LIU, Chongyi, Shanghai 201206 (CN); TANG, Xiaojiao, Shanghai 201206 (CN); SUN, Haiwei, Shanghai 201206 (CN); KUANG, Jingwen, Shanghai 201206 (CN); WANG, Qin, Shanghai 201206 (CN); ZHU, Lingjian, Shanghai 201206 (CN); JIANG, Jun, Shanghai 201206 (CN); HUANG, Jian, Shanghai 201206 (CN); NING, Wei, Shanghai 201206 (CN); LIAO, Cheng, Shanghai 201206 (CN)
(74) Representative: Dragotti & Associati S.P.A.
(86) International application number: PCT/CN2024/102386
(87) International publication number: WO 2025/002352

(57) **Abstract**

A pharmaceutical composition containing a cationic lipid and the use thereof. Specifically, provided are a pharmaceutical composition comprising a carrier, the carrier comprising a cationic lipid, and the molar percentage of the cationic lipid to the carrier being greater than or equal to 10% and less than 50%.

## Description

### TECHNICAL FIELD

The present disclosure pertains to the field of pharmaceuticals and relates to a pharmaceutical composition comprising a cationic lipid and use thereof.

### BACKGROUND

Nucleic acid-based drugs, such as messenger RNA (mRNA), antisense oligonucleotides, small interfering RNA (siRNA), and plasmids, have vast potential for application. A problem that hinders the advancement of this technology is how to safely and effectively deliver these drugs to target organs and cells within the body.

Currently, nucleic acid drug delivery vectors are categorized into two main types: viral vectors and non-viral vectors. Lipid nanoparticle-mediated nucleic acid drug delivery is a primary method for non-viral delivery vectors.

In gene therapy and vaccine applications, lipid nanoparticles have been shown to be excellent vectors for nucleic acids for treating various diseases. Lipid nanoparticles formed from cationic lipids and other auxiliary lipids such as cholesterol, phospholipids, and PEGylated lipids encapsulate nucleic acids, protecting them from degradation, promoting cellular uptake, and reducing immune responses. In addition, lipid nanoparticles offer other advantages for the delivery of bioactive ingredients into cells, including good targeting, minimal side effects, good stability, efficient transfection, etc. Although lipid nanoparticles have demonstrated advantages in delivery, their safety, efficacy, and specificity remain to be improved. There remains a need to develop improved cationic lipid compositions that facilitate the delivery of therapeutic agents and/or prophylactic agents, such as nucleic acids, to cells.

### SUMMARY

The present disclosure provides a pharmaceutical composition comprising a vector, wherein the vector comprises a cationic lipid, and the cationic lipid comprises the compound represented by formula I or a pharmaceutically acceptable salt thereof; a molar percentage of the cationic lipid to the vector is greater than or equal to 10% and less than 50%:

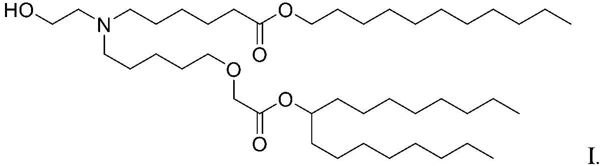

In some embodiments, the cationic lipid in the pharmaceutical composition accounts for 15-49%, including but not limited to 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or a value between any two of these numbers, of a molar amount of the vector.

In some embodiments, the cationic lipid in the pharmaceutical composition accounts for 42-49% of the molar amount of the vector.

In some embodiments, the cationic lipid in the pharmaceutical composition accounts for 45% of the molar amount of the vector.

In some embodiments, the cationic lipid in the pharmaceutical composition accounts for 46% of the molar amount of the vector.

In some embodiments, the cationic lipid in the pharmaceutical composition accounts for 47% of the molar amount of the vector.

In some embodiments, the cationic lipid in the pharmaceutical composition accounts for 48% of the molar amount of the vector.

In some embodiments, the cationic lipid in the pharmaceutical composition accounts for 49% of the molar amount of the vector.

In some other embodiments, the vector in the pharmaceutical composition further comprises a non-cationic lipid, and the non-cationic lipid is selected from the group consisting of at least one of a phospholipid and cholesterol or a derivative thereof.

In some embodiments, the non-cationic lipid in the pharmaceutical composition is selected from the group consisting of a mixture of a phospholipid and cholesterol or a derivative thereof.

A content of the phospholipid in the pharmaceutical composition provided in some embodiments accounts for 5-40%, including but not limited to 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, or a value between any two of these numbers, of the molar amount of the vector. In some embodiments, the content of the phospholipid in the pharmaceutical composition accounts for 15% of the molar amount of the vector.

In some embodiments, the content of the phospholipid in the pharmaceutical composition accounts for 20% of the molar amount of the vector.

In some embodiments, the content of the phospholipid in the pharmaceutical composition accounts for 10-20% of the molar amount of the vector.

In some embodiments, the content of the phospholipid in the pharmaceutical composition accounts for 10% of the molar amount of the vector.

The phospholipid in the pharmaceutical composition provided in some other embodiments is selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and a mixture thereof.

In some embodiments, the phospholipid in the pharmaceutical composition is 1,2-distearoyl-sn-glycero-3-phosphocholine (DSPC).

In some embodiments, the phospholipid in the pharmaceutical composition is 1,2-dioleoyl-sn-glycero-3-phosphoethanolamine (DOPE).

In another aspect, cholesterol or the derivative thereof in the pharmaceutical composition of the present disclosure accounts for 30-60%, including but not limited to 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, or a value between any two of these numbers, of the molar amount of the vector.

In some embodiments, a content of cholesterol or the derivative thereof in the pharmaceutical composition accounts for 35-45% of the molar amount of the vector.

In some embodiments, the content of cholesterol or the derivative thereof in the pharmaceutical composition accounts for 40.5% of the molar amount of the vector.

In some embodiments, the content of cholesterol or the derivative thereof in the pharmaceutical composition accounts for 45% of the molar amount of the vector.

In some other embodiments, cholesterol derivatives include, but are not limited to, sitosterol, ergosterol, campesterol, stigmasterol, brassicasterol, tomatidine, and ursolic acid.

In some embodiments, the non-cationic lipid in the pharmaceutical composition accounts for 35-70%, including but not limited to 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, or a value between any two of these numbers, of the molar amount of the vector.

In some embodiments, the non-cationic lipid in the pharmaceutical composition accounts for 50-65% of the molar amount of the vector.

In some embodiments, the non-cationic lipid in the pharmaceutical composition accounts for 40-55% of the molar amount of the vector.

In some embodiments, the non-cationic lipid in the pharmaceutical composition accounts for 55-62% of the molar amount of the vector.

In another aspect, the molar ratio of cholesterol or the derivative thereof to the phospholipid in the pharmaceutical composition of the present disclosure is 1.0-5.0, including but not limited to 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, or a value between any two of these numbers.

In some embodiments, the molar ratio of cholesterol or the derivative thereof to the phospholipid in the pharmaceutical composition is 2.0-5.0.

In another aspect, the vector in the pharmaceutical composition of the present disclosure further comprises a conjugated lipid, and the conjugated lipid includes, but is not limited to, PEG-modified phosphatidylethanolamine, PEG-modified phosphatidic acid, PEG-modified ceramide, PEG-modified dialkylamine, PEG-modified diacylglycerol, and PEG-modified dialkylglycerol.

In some embodiments, the conjugated lipid in the pharmaceutical composition is selected from the group consisting of distearoylphosphatidylethanolamine polyethylene glycol 2000 (DSPE-PEG2000), dimyristoylglycero-3-methoxypolyethylene glycol 2000 (DMG-PEG2000), and methoxypolyethylene glycol ditetradecylacetamide (ALC-0159). In some embodiments, the conjugated lipid in the pharmaceutical composition is selected from the group consisting of dimyristoylglycero-3-methoxypolyethylene glycol 2000. In some embodiments, the conjugated lipid in the pharmaceutical composition is selected from the group consisting of distearoylphosphatidylethanolamine polyethylene glycol 2000.

In some embodiments, the conjugated lipid inhibits vector (particle) aggregation. In some embodiments, a content of the conjugated lipid in the pharmaceutical composition accounts for 0.5-4%, including but not limited to 0.5%, 0.6%, 0.7%, 0.8%, 0.9%, 1.0%, 1.2%, 1.4%, 1.6%, 1.8%, 2.0%, 2.2%, 2.4%, 2.6%, 2.8%, 3.0%, 3.2%, 3.4%, 3.6%, 3.8%, 4.0%, or a value between any two of these numbers, of the molar amount of the vector. In some embodiments, the content of the conjugated lipid in the pharmaceutical composition accounts for 0.5-1.0% of the molar amount of the vector.

In some embodiments, the content of the conjugated lipid in the pharmaceutical composition accounts for 1.5-2.5% of the molar amount of the vector.

In some embodiments, the content of the conjugated lipid in the pharmaceutical composition accounts for 1-2% of the molar amount of the vector.

In some embodiments, the content of the conjugated lipid in the pharmaceutical composition accounts for 1.5% of the molar amount of the vector.

In some embodiments, the content of the conjugated lipid in the pharmaceutical composition accounts for 2.0% of the molar amount of the vector.

The pharmaceutical composition of the present disclosure further comprises an active agent, and the active agent is selected from the group consisting of nucleic acids, including ribonucleic acids or deoxyribonucleic acids.

In some embodiments, the active agent is selected from the group consisting of any one or any combination of a small interfering RNA (siRNA), a microRNA (miRNA), a small hairpin RNA (shRNA), a messenger RNA (mRNA), a circular RNA, and a self-replicating RNA.

In some embodiments, the active agent is selected from the group consisting of an mRNA. In some embodiments, the mRNA comprises an ORF, and the ORF expresses a protein or polypeptide.

In some embodiments, the mRNA encodes a protein or polypeptide.

In some embodiments, the mRNA encodes an antigen.

In some embodiments, the protein or polypeptide encoded by the mRNA is derived from an infectious agent, or is an infectious agent antigen.

In some embodiments, the protein or polypeptide is a viral antigen and/or a bacterial antigen.

In some embodiments, the virus is selected from the group consisting of adenoviruses, herpesviruses (e.g., herpes simplex virus type I, herpes simplex virus type II, and human herpesvirus type 8), encephalitis viruses, papillomaviruses, varicella zoster virus (VZV), Epstein-Barr virus, human cytomegalovirus, human papillomavirus, BK virus, JC virus, smallpox, poliovirus, hepatitis viruses (e.g., hepatitis A, B, C, D, or E virus), human bocaviruses, parvoviruses (e.g., B19), human astroviruses, Norwalk virus, coxsackieviruses, rhinoviruses, severe acute respiratory syndrome virus, yellow fever virus, dengue virus, West Nile virus, rubella virus, human immunodeficiency viruses (HIV), influenza A or B virus, Guanarito virus, Junin virus, Lassa virus, Machupo virus, Sabia virus, Crimean-Congo hemorrhagic fever virus, ebolaviruses, Marburg virus, measles virus, mumps virus, parainfluenza viruses, respiratory syncytial virus (RSV), human metapneumovirus, Hendra virus, Nipah virus, rabies virus, rotaviruses, circoviruses, Colorado tick fever virus, Hantaan virus, Middle East respiratory coronavirus, Chikungunya virus, and Banna virus.

In some embodiments, the virus is selected from the group consisting of an influenza virus (e.g., influenza A virus or influenza B virus), a coronavirus, and a combination thereof. For example, the coronavirus is SARS-COV-2; the coronavirus antigen is a spike protein; the spike protein is selected from the group consisting of a spike protein of any one of the viral strain SARS-COV-2, SARS-COV-2 Alpha, SARS-COV-2 Beta, SARS-COV-2 Gamma, SARS-COV-2 Kappa, SARS-COV-2 Delta, or SARS-COV-2 Omicron. For example, the influenza virus is selected from the group consisting of influenza A virus and influenza B virus; the influenza virus antigen is a hemagglutinin protein (HA) and/or a neuraminidase (NA); the influenza virus antigen is selected from the group consisting of a hemagglutinin protein and/or a neuraminidase of any one of the viral strains influenza A virus H1N1, influenza A virus H3N2, influenza B virus Victoria, and influenza B virus Yamagata.

In some embodiments, the virus is RSV. For example, the RSV antigen is an RSV attachment protein (G) or an immunogenic fragment thereof, an RSV fusion (F) glycoprotein or an immunogenic fragment thereof, or a combination thereof; for example, the RSV antigen is a pre-fusion glycoprotein F. The RSV antigenic polypeptides or immunogenic fragments thereof and sequences thereof in WO2017070622A and the pre-fusion glycoproteins F and sequences thereof in WO2014160463A, WO2012158613A, WO2017109629A, and WO2017172890A are incorporated herein by reference in their entirety.

In some embodiments, the virus is VZV. For example, the VZV antigen is a VZV glycoprotein. For example, the VZV glycoprotein may be a VZV gE, gI, gB, gH, gK, gL, gC, gN, or gM polypeptide or an immunogenic fragment or epitope thereof. In some embodiments, the VZV glycoprotein is a VZV gE protein, e.g., a wild-type VZV gE protein or a truncation, fragment, or mutant thereof. In some embodiments, the wild-type VZV gE protein is from an Oka strain. In some embodiments, the wild-type VZV gE protein comprises (or is) the amino acid sequence set forth in SEQ ID NO: 1 and/or comprises (or is) the nucleotide sequence set forth in SEQ ID NO: 2.

In some embodiments, the truncation may be any truncated polypeptide, for example, a truncated VZV gE polypeptide comprising amino acids 1 to 200, 1 to 250, 1 to 300, 1 to 350, 1 to 400, 1 to 450, 1 to 500, 1 to 540, 1 to 544, 1 to 545, 1 to 546, 1 to 550, 1 to 560, 1 to 561, 1 to 570, 1 to 573, 1 to 574, or 1 to 575. In some embodiments, the VZV gE protein further comprises point mutation Y569A. The VZV antigenic polypeptides and sequences thereof in WO2017070601A are incorporated herein by reference in their entirety.

In some embodiments, the amino acid sequence of the truncation is the amino acids at positions 1-544, 1-546, 1-561, or 1-574 of the wild-type gE protein. In some embodiments, the amino acid sequence of the truncation comprises, on the basis of the amino acids at positions 1-574 of the wild-type gE protein, mutation Y569A.

In some embodiments, the amino acid sequence of the VZV gE protein is set forth in any one of SEQ ID NO: 1 or SEQ ID NO: 21-SEQ ID NO: 25 or an amino acid sequence having at least 80%, 85%, 90%, 95%, 96%, 97%, 98%, 99%, or 100% sequence identity thereto.

In some embodiments, the nucleotide sequence encoding an open reading frame of the VZV gE protein is set forth in any one of SEQ ID NO: 3-SEQ ID NO: 8 or a nucleotide sequence having at least 80%, 85%, 90%, 95%, 96%, 97%, 98%, 99%, or 100% sequence identity thereto.

In some embodiments, the protein or polypeptide is selected from the group consisting of fructose-bisphosphate aldolase A (ALDOA), α-methylacyl-CoA racemase (AMACR), serum amyloid P component (APCS), angiopoietin 1 (ANGPT1), apolipoprotein A-I (APOA1) Milano, apolipoprotein A-I (APOA1) Paris, apolipoprotein A-I (APOA1), argininosuccinate lyase (ASL), artemin (ARTN), arylsulfatase B (ARSB), bactericidal/permeability-increasing protein (rBPI-21), bone morphogenetic protein 2 (BMP2), bone morphogenetic protein 7 (BMP7), branched-chain keto acid dehydrogenase E1 α polypeptide (BCKDHA), colony stimulating factor 2 (granulocyte-macrophage) (GM-CSF), colony stimulating factor 3 (granulocyte) (GCSF), deoxyribonuclease I (DNase 1), erythropoietin (EPO), factor IX, factor VII, factor XI, fibrinogen A (FGA), fibroblast growth factor 18 (FGF18), fibroblast growth factor 23 (FGF23), fibroblast growth factor 7 (FGF7 or KGF), follistatin (FST), fumarylacetoacetate hydrolase (fumarylacetoacetase) (FAH), galactokinase 1 (GALK1), galactosidase α (GLA), glucan (1,4-α-) branching enzyme 1 (GBE1), glycoprotein hormones, α polypeptide (CGA or FSH-α), hemoglobin β (HBB), hepatocyte growth factor (HGF), human growth hormone (hGH), insulin aspart, insulin glargine, insulin glulisine, insulin lispro, interferon β (IFNB), interferon α2 (IFNA2), interleukin 10 (IL-10), interleukin 15 (IL-15), interleukin 7 (IL-7), klotho (KL), lecithin-cholesterol acyltransferase (LCAT), lysosomal acid lipase A cholesterol esterase (LIPA), lipoprotein lipase (LPL), low-density lipoprotein receptor (LDLR), mannosidase α class 2B member 1 (MAN2B1), microsomal triglyceride transfer protein (MTTP), N-acetylglutamate synthase (NAGS), neuregulin 1 (NRG1), ornithine transcarbamylase (OTC), phosphorylase kinase α 2 (liver) (PHKA2), plasminogen (PLAT), septin-4 (ARTS or SEPT4), serpin peptidase inhibitor clade C (antithrombin) member 1 (SERPINC1), serpin peptidase inhibitor clade F (α-2 antiplasmin pigment epithelium derived factor) member 2 (SERPINF2), sirtuin 1 (SIRT1), sirtuin 6 (SIRT6), solute carrier family 16 member 3 (monocarboxylate transporter 4) (SLC16A3), solute carrier family 2 (facilitated glucose transporter) member 1 (SLC2A1 or GLUT1), sortilin 1 (SORT1), thrombopoietin (THPO), transforming growth factor β (TGFB1), tuftelin 1 (TUFT1), tumor protein p53 (TP53), tyrosinase (TYR), UDP glucuronosyltransferase 1 family polypeptide A1 (UGT1A1), vascular endothelial growth factor (VEGF), X-linked inhibitor of apoptosis (XIAP), and any combination thereof. The mRNA-encoded target polypeptides and sequences thereof in WO2013151736A are incorporated herein by reference in their entirety.

In some embodiments, the protein or polypeptide is a cancer antigen. The cancer antigen includes, but is not limited to, one or more traditional cancer antigens or subject-specific cancer antigens (including RNA encoding one or more known cancer antigens specific for the tumor or cancer antigens specific for each subject; the cancer antigens are referred to as neo-epitopes or subject-specific epitopes or antigens). In some specific embodiments, the cancer antigen is a subject-specific cancer antigen. In some specific embodiments, the subject-specific cancer antigen may represent an exome of a tumor sample of the subject, or a transcriptome of a tumor sample of the subject. In some embodiments, the subject-specific cancer antigens may represent an exosome of the subject. The subject-specific cancer antigens and identification methods therefor described in WO2012159754A and WO2017070618A are incorporated herein by reference in their entirety. In some specific embodiments, the cancer antigen is a traditional cancer antigen. In some embodiments, the traditional cancer antigen is a non-mutated antigen. In some specific embodiments, the traditional cancer antigen is a mutated antigen. For example, cancer antigens may be carcinoembryonic antigen, α1-fetoprotein, isoferritin, fetal sulphoglycoprotein, α2-H-ferritin, and γ-fetoprotein.

In some embodiments, the protein or polypeptide is an antibody or an antigen-binding fragment thereof. For example, the protein or polypeptide is an anti-PD-1 antibody or an antigen-binding fragment thereof. In some specific embodiments, the antibody or the antigen-binding fragment thereof is, for example, a camelid antibody, a chimeric antibody, a humanized antibody, or a fully human antibody, or an antigen-binding fragment thereof. In some specific embodiments, the antibody or the antigen-binding fragment thereof is, for example, a recombinant antibody or a fragment thereof. In some specific embodiments, the antibody or the antigen-binding fragment thereof is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, the mRNA comprises:
(a) an open reading frame (ORF),
(b) an untranslated region element (UTR), and
(c) a polyadenylic acid (poly-A) tail,
wherein (b) the untranslated region element (UTR) may comprise (b1) a 5'UTR and/or (b2) a 3'UTR;
(b) the untranslated region element (UTR) and (c) the polyadenylic acid (poly-A) tail may be present simultaneously or absent simultaneously, or either (b) or (c) is present.

In some embodiments, the mRNA further comprises a 5'Cap structure.

In some embodiments, in the 5'-to-3' direction, the RNA molecule according to any one of the above comprises any one of i)-v):
i) a 5'UTR and an open reading frame (ORF),
ii) an open reading frame (ORF) and a 3'UTR,
iii) a 5'UTR, an open reading frame (ORF), and a 3'UTR,
iv) a 5'UTR, an open reading frame (ORF), a 3'UTR, and a poly-A tail, and
v) a 5'Cap structure, a 5'UTR, an open reading frame (ORF), a 3'UTR, and a poly-A tail, wherein the ORF and the 5'UTR and/or the 3'UTR are derived from the same gene and/or different genes.

In some embodiments, the 5'UTR is selected from the group consisting of a β-globin 5'UTR, a 5'UTR containing a strong Kozak translational initiation signal, a cytochrome b-245α polypeptide (CYBA) 5'UTR, a hydroxysteroid (17-β) dehydrogenase (HSD17B4) 5'UTR, a tobacco etch virus (TEV) 5'UTR, a heat shock protein 70 (Hsp70) 5'UTR, an eIF4G 5'UTR, an ACTG1 5'UTR, a CTSB 5'UTR, and a 5'UTR of an ARHGAP15 gene. In some embodiments, the 5'UTR is derived from or is a 5'UTR sequence of an ACTG1 or ARHGAP15 gene or a derivative sequence thereof. In some embodiments, the ARHGAP15 gene is derived from any species, such as human ARHGAP15, baboon ARHGAP15, or mouse ARHGAP15.

In some embodiments, the 5'UTR is derived from or is a 5'UTR sequence of the human ARHGAP15 gene or a derivative sequence thereof. In some embodiments, the 5'UTR of the ARHGAP15 gene comprises or is the nucleotide sequence set forth in SEQ ID NO: 9 or a nucleotide sequence having at least 80%, 85%, 90%, 95%, 96%, 97%, 98%, 99%, or 100% identity thereto.

In some embodiments, the 3'UTR is selected from the group consisting of an α-globin 3'UTR, a β-globin (hemoglobin subunit beta, HBB) 3'UTR, a β-actin 3'UTR, a CYBA 3'UTR, an albumin 3'UTR, a growth hormone (GH) 3'UTR, a VEEV 3'UTR, a hepatitis B virus (HBV) 3'UTR, an ACTG1 3'UTR, a CTSB 3'UTR, and a 3'UTR of an ARHGAP15 gene.

In some embodiments, the 3'UTR is derived from or is a 3'UTR sequence of an HBB, CTSB, or ARHGAP15 gene or a derivative sequence thereof.

In some embodiments, the 3'UTR comprises or is the nucleotide sequence set forth in SEQ ID NO: 10 or a nucleotide sequence having at least 80%, 85%, 90%, 95%, 96%, 97%, 98%, 99%, or 100% identity thereto. In some embodiments, the poly-A tail is selected from the group consisting of A120, A30L70, HGH polyA, SV40polyA, BGH polyA, rbGlob polyA, and SV40late polyA.

In some specific embodiments, the poly-A tail is selected from the group consisting of 120A and A30L70, wherein the sequence of the A30L70 is set forth in SEQ ID NO: 11, and the sequence of the 120A is set forth in SEQ ID NO: 12.

In some specific embodiments, the nucleotide sequence of the RNA molecule is set forth in any one of SEQ ID NO: 13-SEQ ID NO: 20. In some embodiments, the mRNA comprises modified nucleotides or nucleosides. The modified nucleotides and nucleosides may be naturally occurring modified nucleotides and nucleosides or non-naturally occurring modified nucleotides and nucleosides. The modifications can include those at the sugar, backbone, or nucleobase portion of the nucleotide and/or nucleoside that are recognized in the art. In some specific embodiments, modified nucleobases in the mRNA comprise 1-methyl-pseudouridine (m1ψ), 1-ethyl-pseudouridine (e1ψ), 5-methoxy-uridine (mo5U), 5-methyl-cytidine (m5C), and/or pseudouridine (ψ). In some specific embodiments, modified nucleobases in the mRNA comprise 5-methoxymethyluridine, 5-methylthiouridine, 1-methoxymethylpseudouridine, 5-methylcytidine, and/or 5-methoxycytidine. In some specific embodiments, the mRNA comprises a combination of at least two (e.g., 2, 3, 4, or more) of any of the aforementioned modified nucleobases, including but not limited to chemical modifications.

In some specific embodiments, the 5'Cap structure is located upstream of the 5'UTR, e.g., at the 5' end of the 5'UTR. In some specific embodiments, the 5'Cap structure is a cap structure known to those skilled in the art, such as Cap0 (methylation of the first nucleobase, e.g., m7GpppN), Cap1 (additional methylation of the ribose of a nucleotide adjacent to m7GpppN, e.g., m7G(5')ppp(5')(2'OMeA)pG), Cap2 (additional methylation of the ribose of the 3rd nucleotide downstream of m7GpppN), Cap3 (additional methylation of the ribose of the 3rd nucleotide downstream of m7GpppN), Cap4 (additional methylation of the ribose of the 4th nucleotide downstream of m7GpppN), ARCA (anti-reverse cap analog), modified ARCA (e.g., phosphorothioate-modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In some embodiments, the pharmaceutical composition comprises a cationic lipid, and the cationic lipid comprises the compound represented by formula I or a pharmaceutically acceptable salt thereof; a molar percentage of the cationic lipid to the vector is 40-49%:

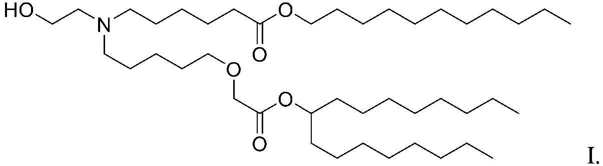

In some embodiments, the pharmaceutical composition comprises:
a) an active agent;
b) a cationic lipid comprising the compound represented by formula I or a pharmaceutically acceptable salt thereof, wherein a molar percentage of the cationic lipid to the vector is greater than or equal to 10% and less than 50%: 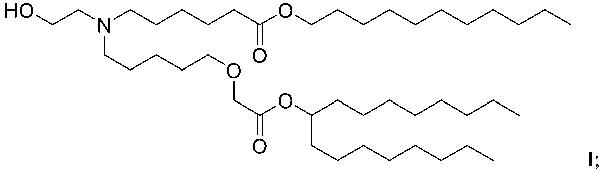
c) a non-cationic lipid selected from the group consisting of a phospholipid and cholesterol or a derivative thereof, wherein the phospholipid accounts for 5-40% of the molar amount of the vector, and cholesterol or the derivative thereof accounts for 30-60% of the molar amount of the vector;
and d) a conjugated lipid, wherein the conjugate accounts for 0.5-4% of the molar amount of the vector.

In some embodiments, the pharmaceutical composition comprises:
a) an active agent comprising an mRNA;
b) a cationic lipid comprising the compound represented by formula I or a pharmaceutically acceptable salt thereof, wherein a molar percentage of the cationic lipid to the vector is greater than or equal to 10% and less than 50%: 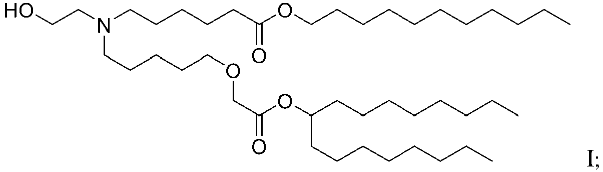
c) a non-cationic lipid selected from the group consisting of the phospholipid DSPC and cholesterol, wherein the phospholipid DSPC accounts for 5-40% of the molar amount of the vector, and cholesterol accounts for 30-60% of the molar amount of the vector;
and d) a conjugated lipid, wherein the conjugate accounts for 0.5-4% of the molar amount of the vector.

In some embodiments, the pharmaceutical composition comprises:
a) an active agent comprising an mRNA;
b) a cationic lipid comprising the compound represented by formula I or a pharmaceutically acceptable salt thereof, wherein a molar percentage of the cationic lipid to the vector is greater than or equal to 10% and less than 50%: 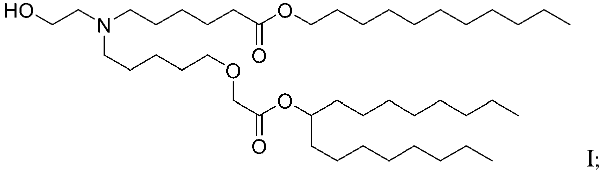
c) a non-cationic lipid selected from the group consisting of the phospholipid DSPC and cholesterol, wherein the phospholipid DSPC accounts for 5-40% of the molar amount of the vector, and cholesterol accounts for 30-60% of the molar amount of the vector;
and d) the conjugated lipid DMG-PEG, wherein the conjugate accounts for 0.5-2% of the molar amount of the vector.

In some embodiments, the pharmaceutical composition comprises:
a) an active agent comprising an mRNA;
b) a cationic lipid comprising the compound represented by formula I or a pharmaceutically acceptable salt thereof, wherein a molar percentage of the cationic lipid to the vector is 40-49%: 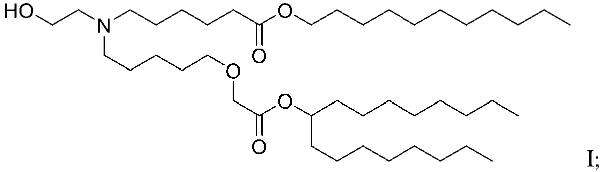
c) a non-cationic lipid selected from the group consisting of the phospholipid DSPC and cholesterol, wherein the phospholipid DSPC accounts for 10-15% of the molar amount of the vector, and cholesterol accounts for 35-42% of the molar amount of the vector;
and d) the conjugated lipid DMG-PEG, wherein the conjugate accounts for 1-2% of the molar amount of the vector.

In some embodiments, the pharmaceutical composition comprises:
a) an active agent comprising an mRNA;
b) a cationic lipid comprising the compound represented by formula I or a pharmaceutically acceptable salt thereof, wherein a molar percentage of the cationic lipid to the vector is 45-49%: 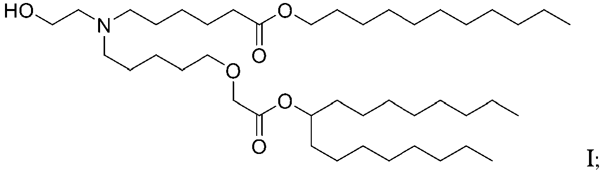
c) a non-cationic lipid selected from the group consisting of the phospholipid DSPC and cholesterol, wherein the phospholipid DSPC accounts for 10-15% of the molar amount of the vector, and cholesterol accounts for 35-42% of the molar amount of the vector;
and d) the conjugated lipid DMG-PEG, wherein the conjugate accounts for 1-2% of the molar amount of the vector.

In another aspect, the pharmaceutical composition of the present disclosure is a nanoparticle formulation, and the nanoparticle formulation has an average particle size of 10 nm-220 nm. In some embodiments, the nanoparticle formulation has an average particle size of 100-150 nm. In some embodiments, the nanoparticle formulation has an average particle size of 150-200 nm. In some embodiments, the nanoparticle formulation has an average particle size of 80-160 nm. In some embodiments, the nanoparticle formulation has an average particle size of 80-150 nm. In some embodiments, the nanoparticle formulation has an average particle size of 70-160 nm.

In some embodiments, the average particle size of the nanoparticles is determined by a dynamic light scattering method.

The present disclosure further provides a freeze-dried formulation obtained by freeze-drying the aforementioned pharmaceutical composition. In some embodiments, the freeze-drying comprises a freezing step, a primary drying step, and a secondary drying step. In some embodiments, the freeze-drying of the present disclosure is performed with reference to the method in CN107567497A, and the relevant contents are incorporated herein by reference for illustrative purposes.

The present disclosure further provides a reconstituted solution obtained by a step of reconstituting the aforementioned freeze-dried formulation, wherein a solvent for the reconstitution is water for injection.

The present disclosure further provides a preparation method for the aforementioned pharmaceutical composition, freeze-dried formulation, or reconstituted solution, the method comprising a step of mixing the cationic lipid, the non-cationic lipid, and the conjugated lipid in an organic solvent. In some embodiments, the organic solvent is selected from the group consisting of ethanol.

In some embodiments, the pharmaceutical composition is prepared with reference to the method in journal literature (Pharm. Res. 22(2005) 362-372) or US9504651, and the relevant contents are incorporated herein by reference for illustrative purposes.

The present disclosure further provides use of the aforementioned pharmaceutical composition, freeze-dried formulation, or reconstituted solution in the manufacture of a medicament for inducing a protective immune response in a vertebrate.

The present disclosure further provides use of the aforementioned pharmaceutical composition, freeze-dried formulation, or reconstituted solution in the manufacture of a medicament for treating a disease or dysfunction in a mammal. In some embodiments, the disease is preferably selected from the group consisting of a viral infection (e.g., a disease caused by a coronavirus, an influenza virus, or an HIV), a liver disease, cancer, and herpes.

### Terminology

The term "lipid" refers to a group of organic compounds that include, but are not limited to, esters of fatty acids and are characterized by being insoluble in water but soluble in many organic solvents. They are usually divided into at least three categories: (1) "simple lipids", which include fats and oils as well as waxes; (2) "compound lipids", which include phospholipids and glycolipids; and (3) "derived lipids" such as steroids.

The term "cationic lipid" refers to any one of a number of lipid species that carry a net positive charge at a selected pH, such as a physiological pH (e.g., a pH of about 7.0).

The term "anionic lipid" refers to any lipid that is negatively charged at a physiological pH. These lipids include, but are not limited to, phosphatidylglycerol, cardiolipin, diacylphosphatidylserine, diacylphosphatidic acid, N-dodecanoylphosphatidylethanolamine, N-succinylphosphatidylethanolamine, N-glutarylphosphatidylethanolamine, lysylphosphatidylglycerol, palmitoyloleyolphosphatidylglycerol (POPG), and other anionic modifying groups linked to neutral lipids.

The term "neutral lipid" refers to any one of a number of lipid species that exist either in an uncharged or neutral zwitterionic form at a selected pH. At a physiological pH, such lipids include, for example, diacylphosphatidylcholine, diacylphosphatidylethanolamine, ceramides, sphingomyelin, cephalin, cholesterol, cerebrosides, and diacylglycerol.

The term "amphiphilic lipid" refers, in part, to any suitable material in which the hydrophobic portion of the lipid material is oriented into a hydrophobic phase and the hydrophilic portion is oriented into an aqueous phase. The hydrophilic nature results from the presence of polar or charged groups such as sugars, phosphate ions, carboxyl, sulfate ions, amino, sulfhydryl, nitro, hydroxy, and other similar groups. Hydrophobicity can be conferred by the inclusion of apolar groups that include, but are not limited to, long-chain saturated and unsaturated aliphatic hydrocarbon groups and such groups substituted with one or more aromatic, alicyclic, or heterocyclyl groups. Examples of amphiphilic compounds include, but are not limited to, phospholipids, aminolipids, and sphingolipids. The term "non-cationic lipid" refers to any amphiphilic lipid and any other neutral or anionic lipid.

The term "conjugated lipid" refers to a conjugated lipid that inhibits the aggregation of lipid particles. The conjugated lipid includes, but is not limited to, PEG-modified phosphatidylethanolamine, PEG-modified phosphatidic acid, PEG-modified ceramide, PEG-modified dialkylamine, PEG-modified diacylglycerol, and PEG-modified dialkylglycerol.

The term "mammal" refers to any mammalian species such as humans, mice, rats, dogs, cats, hamsters, guinea pigs, rabbits, and livestock.

"Active agent" refers to any agent that, when administered to a subject, has a therapeutic, diagnostic, and/or prophylactic effect and/or elicits a desired biological and/or pharmacological effect. Active agents are also referred to as "active substances" or "therapeutic agents". Such agents include, but are not limited to, cytotoxins, radioactive ions, chemotherapeutic agents, small-molecule drugs, proteins, and nucleic acids.

The term "nucleic acid" as used herein refers to a polymer containing at least two deoxyribonucleotides or ribonucleotides in a single-stranded or double-stranded form and includes DNA and RNA. The term "nucleic acid molecule" as used herein is synonymous with "nucleic acid" and "polynucleotide". DNA may be in the form of, e.g., antisense molecules, plasmid DNA, pre-concentrated DNA, PCR products, vectors (P1, PAC, BAC, YAC, artificial chromosomes), expression cassettes, chimeric sequences, chromosomal DNA, or derivatives and combinations of these groups. RNA may be in the form of small interfering RNA (siRNA), microRNA (miRNA), small hairpin RNA (shRNA), messenger RNA (mRNA), circular RNA, or self-replicating RNA.

"Messenger RNA" (mRNA) refers to any polynucleotide that encodes a (at least one) polypeptide (a naturally occurring, non-naturally occurring, or modified polymer of amino acids) and can be translated to produce the encoded polypeptide *in vitro, in vivo,* or *ex vivo.* Those skilled in the art will appreciate that, unless otherwise specified, the polynucleotide sequences described in the present application will represent "T" in a representative DNA sequence, but where the sequence represents RNA (e.g., mRNA), "T" will be substituted with "U". Thus, any RNA polynucleotide encoded by a DNA identified by a particular sequence identification number may also comprise the corresponding RNA (e.g., mRNA) sequence encoded by the DNA, wherein each "T" of the DNA sequence is substituted with "U". The basic components of an mRNA molecule typically include at least one coding region, a 5' untranslated region (UTR), a 3'UTR, a 5'-end cap, and a polyadenylic acid tail (poly-A tail). The polynucleotides of the present disclosure may function as mRNA but are distinguished from wild-type mRNA in their functional and/or structural design features, which serve to overcome existing problems of effective polypeptide expression using nucleic acid-based treatments.

The term "antigen" encompasses any substance that will elicit an immune response. In particular, "antigen" relates to any substance, preferably a peptide or protein that specifically reacts with antibodies or T lymphocytes (T cells). In the present disclosure, the term "antigen" includes any molecule that comprises at least one epitope. Preferably, an antigen herein is a molecule that (optionally after processing) induces an immune response that is preferably specific for the antigen (including cells that express the antigen). Herein, any suitable antigen that is a candidate for an immune response may be used, wherein the immune response is preferably a cellular immune response. In the context of some embodiments, the antigen is preferably presented by cells, preferably by antigen-presenting cells (including diseased cells, particularly cancer cells, in the context of MHC molecules), which results in an immune response against the antigen. Preferably, the antigen is a product corresponding to or derived from a natural antigen. Such natural antigens include tumor antigens.

The term "cancer antigen" is used interchangeably with "tumor antigen" or "tumor-associated antigen"; it is capable of stimulating an immune response, preferably a cellular response against the antigen or cells characterized by expressing the antigen and preferably presenting the antigen (e.g., diseased cells, particularly cancer cells). In some embodiments, "cancer antigen" is specifically expressed under normal conditions in a limited number of tissues and/or organs or in specific developmental stages (for example, the tumor antigen may be specifically expressed under normal conditions in gastric tissues (preferably in the gastric mucosa), in reproductive organs (e.g., in a testis), in trophoblastic tissues (e.g., in the placenta), or in germline cells), and is expressed or abnormally expressed in one or more tumor or cancer tissues. In some embodiments, "a limited number" means being not more than 3, e.g., not more than 2.

"Polypeptide" and "protein" are used interchangeably, refer to a polymer of amino acid residues, and are suitable for naturally occurring amino acid polymers and non-naturally occurring amino acid polymers. Unless otherwise stated, a particular polypeptide sequence also implicitly encompasses conservatively modified variants thereof.

The term "varicella-zoster virus (VZV)" refers to an α herpesvirus that exists in the form of a spherical multilayered structure. The viral genome is surrounded by a protein capsid structure covered by an amorphous layer of tegument proteins. The tegument, which consists of virally encoded proteins and enzymes, is located in the space between the nucleocapsid and the viral envelope. The viral envelope is obtained from host cell membranes and contains virally encoded glycoproteins.

The term "VZV glycoprotein" refers to the encoded glycoproteins gE, gI, gB, gH, gK, gL, gC, gN, and gM, which function in different steps of the viral replication cycle. The most abundant glycoprotein found in infected cells, as well as in mature virions, is glycoprotein E (gE, ORF 68), which is a major component of the virion envelope and is essential for viral replication. Glycoprotein I (gI, ORG 67) forms a complex with gE in infected cells, which facilitates the endocytosis of the glycoproteins and directs them to the trans-Golgi network (TGN), where the final viral envelope is obtained. Glycoprotein I (gI) is required in the TGN for VZV envelopment and for effective membrane fusion during VZV replication. VZV gE and gI are found complexed together on the surface of infected host cells. Glycoprotein B (ORF 31), which is the second most prevalent glycoprotein and is thought to play a role in virus entry, binds to neutralizing antibodies. Glycoprotein H is thought to have a fusion function facilitating the cell-to-cell spread of the virus.

Antibodies to gE, gB, and gH are prevalent after natural infection and after vaccination and have been shown to neutralize viral activity *in vitro.* An exemplary gE protein amino acid sequence is set forth in any one of SEQ ID NO: 1 or SEQ ID NO: 21-SEQ ID NO: 25 of the present disclosure.

The term "mutant" of a wild-type VZV gE protein, "mutant" of a VZV gE protein, or "VZV gE protein mutant" refers to a polypeptide that displays introduced mutations relative to the wild-type VZV gE protein and has immunogenicity against the wild-type VZV gE protein.

The term "mutation" refers to deletion, addition, or substitution of amino acid residues in the amino acid sequence of a protein or polypeptide compared to the amino acid sequence of a reference protein or polypeptide. Throughout the specification and claims, the substitution of an amino acid at one particular position in a protein sequence is referred to using the notation "(amino acid residue in wild type protein)(amino acid position)(amino acid residue in engineered protein)". For example, the notation Y75A refers to the substitution of a tyrosine (Y) residue at position 75 of the amino acid sequence of the reference protein with an alanine (A) residue (in a mutant of the reference protein). Where there is a variation in the amino acid residue at the same position among different wild-type sequences, the amino acid code preceding the position number may be omitted from the notation, such as "75A".

"Codon optimization" refers to the replacement of a codon in the target sequence that is rarely seen in highly expressed genes of a given species with a codon commonly seen in highly expressed genes of the species, with the codons before and after the replacement encoding the same amino acid. Various species may exhibit specific usage biases for certain codons for a particular amino acid. The codon usage bias (the difference in codon usage between organisms) is often correlated with the translation efficiency of messenger RNA (mRNA), which in turn is believed to particularly depend on the characteristics of the codons translated and the availability of specific transfer RNA (tRNA) molecules. The predominance of a selected tRNA in a cell is typically a reflection of the codons most frequently used in peptide synthesis. Thus, on the basis of codon optimization, genes can be modified for the optimal gene expression in a given organism. Thus, the option of optimal codons depends on the codon usage bias of the host genome.

As is known in the art, the term "identity" or "homology" refers to a relationship between the sequences of two or more polypeptides or polynucleotides as determined by comparing the sequences. In the art, identity also means the degree of sequence relatedness between them as determined by the number of matches between strings of two or more amino acid residues or nucleic acid residues. Identity measures the percentage of identical matches between the smaller of two or more sequences with gap alignments (if any) proposed by a particular mathematical model or computer program (e.g., "algorithm"). "Identity%", when applied to polypeptide or polynucleotide sequences, is defined as the percentage of residues (amino acid residues or nucleic acid residues) in the candidate amino acid or nucleic acid sequence that are identical to the residues in the amino acid sequence or nucleic acid sequence of a second sequence after the sequences are aligned and gaps are introduced, if necessary, to achieve the maximum percent identity. Methods and computer programs for alignment are well known in the art. It will be appreciated that identity depends on the calculation of percent identity but may differ in value due to gaps and penalties introduced in the calculation. Generally, variants of a particular polynucleotide or polypeptide have at least 40%, 45%, 50%, 55%, 60%, 65%, 70%, 75%, 80%, 85%, 90%, 91%, 92%, 93%, 94%, 95%, 96%, 97%, 98%, or 99% but less than 100% sequence identity to that particular reference polynucleotide or polypeptide as determined by sequence alignment programs and parameters described herein and known to those skilled in the art. Such tools for alignment include those of the BLAST kit (Stephen F. Altschul, et al. (1997), "Gapped BLAST and PSI-BLAST: a new generation of protein database search programs", Nucleic Acids Res. 25:3389-3402). Another popular local alignment technique is based on the Smith-Waterman algorithm (Smith, T. F. and Waterman, M. S. (1981) "Identification of common molecular subsequences." J. Mol. Biol. 147:195-197). A general global alignment technique based on dynamic programming is the Needleman-Wunsch algorithm (Needleman, S. B. and Wunsch, C. D. (1970) "A general method applicable to the search for similarities in the amino acid sequences of two proteins." J. Mol. Biol. 48:443-453). Recently, a fast optimal global sequence alignment algorithm (FOGSAA) has been developed. The algorithm is said to produce global alignment of nucleotide and protein sequences faster than other optimal global alignment methods (including the Needleman-Wunsch algorithm). Other tools are described herein, especially in the definition of "identity" below.

The term "vaccine" relates to a pharmaceutical preparation (pharmaceutical composition) or product that, upon administration, induces an immune response, in particular a cellular immune response, that recognizes and attacks pathogens (including viruses or bacteria) or diseased cells such as cancer cells. A vaccine can be used to prevent or treat a disease, for example, to prevent a viral infection or to treat cancer.

The term "5' untranslated region element" (5'UTR) refers to an mRNA region that is directly upstream (i.e., 5') of the start codon (i.e., the first codon of an mRNA transcript translated by a ribosome) and does not encode a polypeptide.

The term "3' untranslated region element" (3'UTR) refers to an mRNA region that is directly downstream (i.e., 3') of the stop codon (i.e., the codon of an mRNA transcript that signals translation termination) and does not encode a polypeptide.

The term "open reading frame" (ORF) refers to a continuous stretch of DNA that begins with a start codon (e.g., methionine (ATG)), ends with a stop codon (e.g., TAA, TAG, or TGA), and encodes a protein or polypeptide.

The term "polyadenylic acid (polyA) tail" refers to an mRNA region that is downstream, e.g., directly downstream (i.e., 3'), of a 3'UTR that contains multiple consecutive adenosine monophosphates. A polyadenylic acid tail may contain 10 to 300 adenosine monophosphates. For example, a polyadenylic acid tail may contain 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 adenosine monophosphates. In some embodiments, a polyadenylic acid tail contains 50 to 250 adenosine monophosphates. In a related biological setting (e.g., in cells or *in vivo*), the poly(adenylic acid) tail functions to protect mRNA from enzymatic degradation, e.g., in the cytoplasm, and aids in transcription termination and/or export of the mRNA from the nucleus and translation.

The "Z-average size" described in the present disclosure, e.g., "an average particle size of less than 2000 nm", is an average light intensity value calculated from the light intensities contributed by different types of particles. The "average particle size" of particles can be measured using conventional particle size measurement techniques well known to those skilled in the art. Such techniques include sedimentation field flow fractionation, photon correlation spectroscopy, light scattering, etc.

The polydispersity index (PDI) of the present disclosure reflects the degree of uniformity of the particle size of particles, and is an important index for particle size characterization. The terms "mixed into" and "mixed" mean that the order of addition of the components is not limited. For example, when A is mixed into B, it can mean either A is added to B or B is added to A; when A and B are mixed, it can mean either A is added to B and mixed or B is added to A and mixed.

The numerical values in the present disclosure are instrument measurements and have a certain degree of error. Generally, ±10% is a reasonable margin of error. It is certainly necessary to consider the context in which the numerical values are used; for example, the numerical value of the particle size of the active ingredient after measurement has a margin of error of no greater than ±10%; the margin of error may be ±9%, ±8%, ±7%, ±6%, ±5%, ±4%, ±3%, ±2%, or ±1%, preferably ±5%.

The compound of formula I of the present disclosure is prepared with reference to the method in WO2023125738, and the relevant contents are incorporated herein by reference for illustrative purposes.

The mRNA used for testing in the present disclosure is luciferase mRNA.

### BRIEF DESCRIPTION OF THE DRAWINGS

FIG. 1: luciferase protein expression levels after transfection of HEK293 cells with liposomal nanoparticles corresponding to lipid compositions 1-15.
FIG. 2: the particle size stability of a lipid composition at 4 °C.
FIG. 3: the particle size stability of a lipid composition at 25 °C.
FIG. 4: a VZV mRNA *in vitro* activity expression assay.
FIG. 5: a serum anti-VZV gE IgG assay.

### DETAILED DESCRIPTION

The present disclosure is further described below with reference to examples. However, these examples do not limit the scope of the present disclosure.

Experimental methods without specific conditions indicated in the examples of the present disclosure were generally conducted under conventional conditions, or conditions recommended by the manufacturers of the starting materials or commercial products. Reagents without specific sources indicated were commercially available conventional reagents.

### Example 1

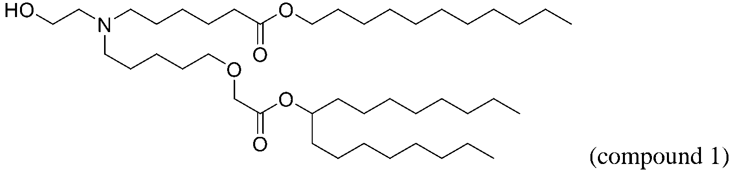

### Step 1)

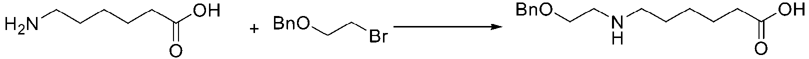

6-Aminocaproic acid (21.5 g, 100 mmol) was dissolved in a mixed solution of ethanol and water (EtOH/H₂O, v/v = 2:1, 200 mL). CsOH·H₂O (4.0 g, 100 mmol) was added, and benzyloxybromoethane (13.1 g, 100 mmol) was added. The mixture was left to react at room temperature for 40 h. The reaction mixture was concentrated to remove most of the ethanol, and the residue was directly purified by reversed-phase column chromatography (acetonitrile/water/0.1% trifluoroacetic acid). The eluate was directly concentrated to dryness under reduced pressure to give a white solid (5.6 g, yield: 21%).

MS: 266.2 [M+H]⁺.

¹H NMR (400 MHz, D₂O) δ 7.36 (s, 5H), 4.53 (s, 2H), 3.72-3.67 (m, 2H), 3.20-3.14 (m, 2H), 2.95-2.88 (m, 2H), 2.10 (t, *J =* 7.3 Hz, 2H), 1.57 (dd, *J =* 15.3, 7.7 Hz, 2H), 1.47 (dd, *J =* 15.0, 7.5 Hz, 2H), 1.30-1.22 (m, 2H).

### Step 2)

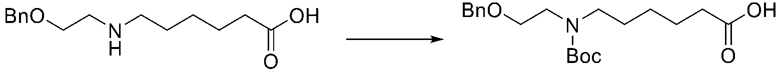

6-((2-(Benzyloxy)ethyl)amino)hexanoic acid (5.6 g, 21.1 mmol) was weighed into a reaction flask. 1,4-Dioxane (80 mL) and an aqueous sodium hydroxide solution (42.2 mL, 1.0 M) were added, and Boc₂O (5.5 g, 25.2 mmol) was added dropwise. The mixture was left to react at room temperature for 1 h, and LC-MS analysis showed that the reaction was complete. The reaction mixture was concentrated to remove the dioxane. The pH of the aqueous phase was adjusted to about 5 with dilute hydrochloric acid, and extraction was performed with ethyl acetate (50 mL × 3). The organic phases were combined, dried, and concentrated to give a nearly colorless gum (7.2 g, yield: 93%). The product was directly used in the next step.

MS: 366.2 [M+H]⁺.

### Step 3)

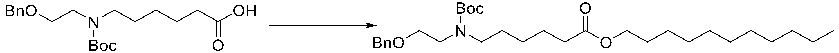

6-((2-(Benzyloxy)ethyl)(tert-butoxycarbonyl)amino)hexanoic acid (6.1 g, 16.7 mmol) was weighed into a reaction flask, and dichloromethane (150 mL) was added. In an ice bath, undecanol (2.73 g, 15.9 mmol), DMAP (408 mg, 3.34 mmol), and DIPEA (4.3 g, 33.4 mmol) were sequentially added, and EDCI (3.84 g, 20.0 mmol) was added. After 10 min of stirring, the ice bath was removed. The mixture was left to react overnight at room temperature, and TLC monitoring showed that the reaction was complete. Water (100 mL) was added to the reaction mixture. The organic phase was separated, washed with dilute hydrochloric acid, water, and sodium bicarbonate in sequence, dried, concentrated, and purified by column chromatography (PE:EtOAc = 20:1-10:1) to give a nearly colorless gum (6.8 g, yield: 82%).

MS: 520.4 [M+H]⁺.

¹H NMR (400 MHz, CDCl₃) δ 7.30-7.20 (m, 5H), 4.44 (s, 2H), 3.98 (t, *J =* 6.7 Hz, 2H), 3.51 (d, *J* = 18.6 Hz, 2H), 3.32 (d, *J =* 21.5 Hz, 2H), 3.16 (s, 2H), 2.21 (t, *J =* 7.5 Hz, 2H), 1.55 (tt, *J =* 13.3, 6.8 Hz, 6H), 1.36 (d, *J =* 8.4 Hz, 9H), 1.21 (d, *J =* 15.5 Hz, 18H), 0.81 (t, *J =* 6.8 Hz, 3H).

### Step 4)

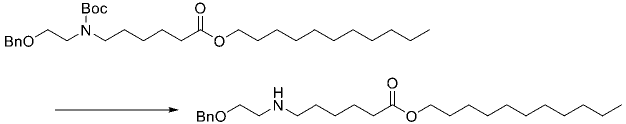

Undecyl 6-((2-(benzyloxy)ethyl)(tert-butoxycarbonyl)amino)hexanoate (7.6 g, 14.6 mmol) was weighed out and dissolved in dichloromethane, and TFA (20 mL) was added. The mixture was left to react at room temperature for 2 h, and the reaction mixture was directly concentrated. The residue was dissolved in dichloromethane (100 mL), and saturated sodium bicarbonate (100 mL) was added. The organic phase was separated, dried, and concentrated to give a gum. The gum was then dissolved in dichloromethane, and HCl/dioxane (4.0 M, 4 mL) was added. The mixture was directly concentrated to give a white solid (5.0 g, yield: 81%).

MS: 420.3 [M+H]⁺.

¹H NMR (400 MHz, CDCl₃) δ 9.51 (s, 2H), 7.39-7.28 (m, 5H), 4.57 (s, 2H), 4.03 (t, *J* = 6.8 Hz, 2H), 3.88 (t, *J* = 4.8 Hz, 2H), 3.18 (s, 2H), 3.03 (d, *J* = 3.4 Hz, 2H), 2.28 (t, *J =* 7.4 Hz, 2H), 2.02 (s, 1H), 1.93-1.84 (m, 2H), 1.61 (dd, *J* = 13.8, 6.7 Hz, 4H), 1.42-1.17 (m, 18H), 0.88 (t, *J* = 6.8 Hz, 3H).

### Step 5)

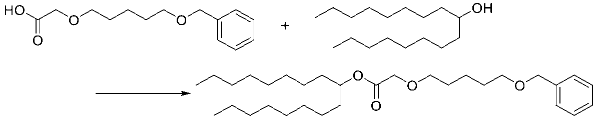

2-((5-(Benzyloxy)pentyl)oxy)acetic acid (prepared with reference to Chemical and Pharmaceutical Bulletin, 1992, vol. 40, #3, p. 617-623) (7.6 g, 30.12 mmol) was weighed into a reaction flask, and dichloromethane (150 mL) was added. In an ice bath, heptadecan-9-ol (prepared with reference to WO2020/219876) (6.18 g, 24.1 mmol) and DMAP (3.68 g, 30.12 mmol) were sequentially added, and EDCI (6.93 g, 36.14 mmol) was added. After 10 min of stirring, the ice bath was removed. The mixture was left to react overnight at room temperature, and TLC monitoring showed that the reaction was complete. Water (100 mL) was added to the reaction mixture. The organic phase was separated, washed with dilute hydrochloric acid, water, and sodium bicarbonate in sequence, dried, concentrated, and purified by column chromatography (PE:EtOAc = 50:1-10:1) to give a nearly colorless gum (10.9 g, yield: 92%).

¹H NMR (400 MHz, CDCl₃) δ 7.36-7.31 (m, 4H), 7.30-7.26 (m, 1H), 4.96 (p, *J =* 6.3 Hz, 1H), 4.50 (s, 2H), 4.04 (s, 2H), 3.52 (t, *J* = 6.6 Hz, 2H), 3.47 (t, *J =* 6.6 Hz, 2H), 1.69-1.61 (m, 4H), 1.56 (d, *J =* 16.5 Hz, 6H), 1.25 (s, 24H), 0.88 (t, *J =* 6.8 Hz, 6H).

### Step 6)

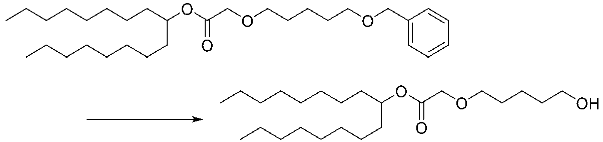

Heptadecan-9-yl 2-((5-(benzyloxy)pentyl)oxy)acetate (4.0 g, 8.15 mmol) was weighed out and dissolved in THF (100 mL). Pd(OH)₂/C (400 mg, 20%) was added, and hydrogen gas was introduced into the system. The mixture was left to react at room temperature for 2 h, and TLC monitoring showed the reaction was complete. The reaction mixture was filtered, and the filtrate was concentrated to give a colorless gum (3.2 g, yield: 98%). The product was directly used in the next step.

¹H NMR (400 MHz, CDCl₃) δ 4.97 (dd, *J =* 12.4, 6.1 Hz, 1H), 4.04 (s, 2H), 3.66 (t, *J* = 6.5 Hz, 2H), 3.54 (t, *J* =6.5 Hz, 2H), 1.71-1.57 (m, 4H), 1.56-1.42 (m, 6H), 1.25 (s, 24H), 0.88 (t, *J =* 6.8 Hz, 6H).

### Step 7)

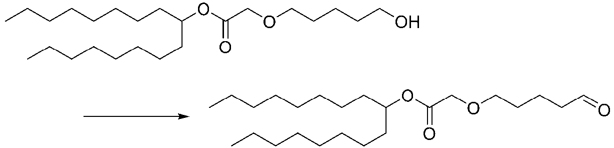

Heptadecan-9-yl 2-((5-hydroxypentyl)oxy)acetate (2.4 g, 6.0 mmol) was weighed out and dissolved in dichloromethane (100 mL). In an ice bath, DMP (3.82 g, 9.0 mmol) was added, and the mixture was left to react at room temperature for 1.5 h. A saturated aqueous sodium thiosulfate solution (100 mL) was added, and the mixture was stirred for 30 min. The organic phase was separated, washed with a saturated aqueous sodium bicarbonate solution (100 mL × 2), dried, and concentrated to give a light yellow gum (2.5 g). The product was directly used in the next step.

¹H NMR (400 MHz, CDCl₃) δ 9.78 (s, 1H), 4.96 (p, *J =* 6.3 Hz, 1H), 4.04 (s, 2H), 3.55 (t, *J =* 6.1 Hz, 2H), 2.49 (td, *J =* 7.2, 1.5 Hz, 2H), 1.81-1.72 (m, 2H), 1.68 (d, *J =* 7.6 Hz, 2H), 1.60-1.48 (m, 4H), 1.26 (s, 24H), 0.88 (t, *J =* 6.8 Hz, 6H).

### Step 8)

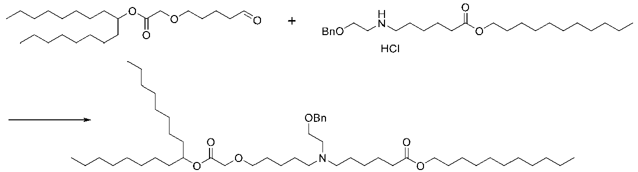

Heptadecan-9-yl 2-((5-oxopentyl)oxy)acetate (2.4 g, 6 mmol) was weighed out and dissolved in dichloromethane (50 mL). Undecyl 6-((2-(benzyloxy)ethyl)amino)hexanoate hydrochloride (2.46 g, 5.4 mmol) was added, and DIPEA (1.16 g, 9.0 mmol) and acetic acid (1.08 g, 18.0 mmol) were added. The mixture was stirred until complete dissolution was achieved. In an ice bath, NaBH(OAc)₃ (3.18 g, 15.0 mmol) was added. The mixture was left to react overnight at room temperature, and TLC monitoring showed that the reaction was complete. Water (100 mL) was added. The organic phase was separated, washed with a saturated aqueous sodium bicarbonate solution (50 mL × 1), dried, concentrated, and purified by column chromatography (PE:EtOAc = 5:1-2:1) to give a colorless liquid (4.1 g, yield: 85%).

¹H NMR (400 MHz, CDCl₃) δ 7.33 (d, *J =* 4.5 Hz, 4H), 7.30-7.26 (m, 1H), 4.99-4.91 (m, 1H), 4.52 (s, 2H), 4.08-4.02 (m, 4H), 3.57-3.47 (m, 4H), 2.68 (t, *J =* 5.8 Hz, 2H), 2.45 (s, 4H), 2.28 (t, *J* = 7.5 Hz, 2H), 1.67-1.57 (m, 6H), 1.48 (dd, *J* = 37.5, 6.0 Hz, 6H), 1.38-1.18 (m, 46H), 0.88 (t, *J =* 6.8 Hz, 9H).

### Step 9)

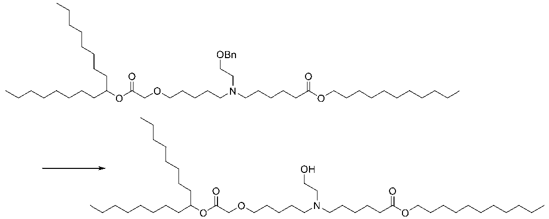

Undecyl 6-((2-(benzyloxy)ethyl)(5-(2-(heptadecan-9-yloxy)-2-oxoethoxy)pentyl)amino)hexanoate (1 g, 1.25 mmol) was weighed into a reaction flask and dissolved in ethanol. Pd(OH)₂/C(500 mg, 20%) was added, and hydrogen gas was introduced into the system. The mixture was left to react overnight at room temperature, and TLC monitoring showed that the reaction was complete. The reaction mixture was filtered, and the filtrate was concentrated to give a colorless gum. Column chromatography purification was performed (CH₂Cl₂:MeOH = 10:1) to give a colorless gum (450 mg, yield: 51%).

¹H NMR (400 MHz, CDCl₃) δ 5.00-4.89 (m, 1H), 4.09-4.01 (m, 4H), 3.61 (s, 2H), 3.52 (t, *J =* 6.4 Hz, 2H), 2.68 (s, 2H), 2.57 (s, 4H), 2.30 (t, *J =* 7.4 Hz, 2H), 1.70-1.59 (m, 6H), 1.53 (s, 8H), 1.43-1.18 (m, 44H), 0.88 (t, *J* = 6.7 Hz, 9H).

### Example 2:

### Preparation of LNP lipid particles

Compound 1 (the compound of formula I), DSPC, cholesterol, and a DMG-PEG 2000 solution were mixed at certain molar percentages (mol%) (the percentage of the molar amount of each component to the total molar amount of the vector) to prepare ethanol lipid solutions. The solutions were prepared from the four components according to the molar ratios shown in Table 1. An mRNA encoding luciferase protein (the nucleotide sequence is set forth in SEQ ID NO: 26) was dissolved in a 50 mM pH 4 acetate buffer to prepare an aqueous mRNA solution. Each of the ethanol lipid solutions and the aqueous mRNA solution were mixed by microfluidics at a volume ratio of 1:3 (the weight ratio of compound 1 to the mRNA was 12:1) to prepare liposomes. The ethanol was removed by dialysis against a 20 mM Tris solution, and the liposomes were finally exchanged into a 20 mM Tris 8% sucrose solution to obtain a liposomal nanoparticle composition in which the mRNA was encapsulated.

### Lipid particle composition characterization

### Characterization method

The particle size and polydispersity index (PDI) of the liposomal nanoparticles were determined using a Malvern Zetasizer Pro in a 173° backscatter detection mode and dynamic light scattering.

The liposome encapsulation efficiency was determined using a Quant-iT RiboGreen RNA assay kit.

**Table 1**

| No. | Compound 1 mol % | DSPC mol % | Cholesterol mol % | PEG-DMG mol % | Particle size nm | PDI | Encapsulation efficiency % |
|---|---|---|---|---|---|---|---|
| 1 | 43.8 | 18.8 | 35.3 | 2.1 | 87.2 | 0.31 | 94 |
| 2 | 45.0 | 13.5 | 40.5 | 1.0 | 113.4 | 0.19 | 94 |
| 3 | 42.6 | 17.6 | 38.1 | 1.8 | 79.8 | 0.22 | 95 |
| 4 | 45.0 | 12.0 | 40.5 | 2.5 | 76.0 | 0.25 | 95 |
| 5 | 43.8 | 15.5 | 39.3 | 1.4 | 92.0 | 0.18 | 94 |
| 6 | 37.0 | 20.0 | 40.5 | 2.5 | 76.5 | 0.22 | 95 |
| 7 | 40.5 | 18.8 | 39.3 | 1.4 | 89.4 | 0.20 | 95 |
| 8 | 45.0 | 20.0 | 34.0 | 1.0 | 85.9 | 0.22 | 95 |
| 9 | 43.8 | 14.8 | 39.3 | 2.1 | 70.7 | 0.15 | 96 |
| 10 | 38.5 | 20.0 | 40.5 | 1.0 | 99.3 | 0.14 | 95 |
| 11 | 39.8 | 18.8 | 39.3 | 2.1 | 70.5 | 0.12 | 96 |
| 12 | 43.8 | 18.8 | 36.0 | 1.4 | 84.1 | 0.18 | 96 |
| 13 | 45.0 | 20.0 | 32.5 | 2.5 | 70.5 | 0.21 | 97 |
| 14 | 48.0 | 10.0 | 40.5 | 1.5 | 90.8 | 0.12 | 97 |
| 15 | 45.0 | 15.0 | 38.5 | 1.5 | 84.1 | 0.15 | 96 |

### Test Example 1: Evaluation of mRNA Delivery Efficiency of Lipid Particle Composition Vectors

The protein expression levels after the mRNA was delivered to cells by liposomal nanoparticles corresponding to lipid compositions 1-15 were determined.

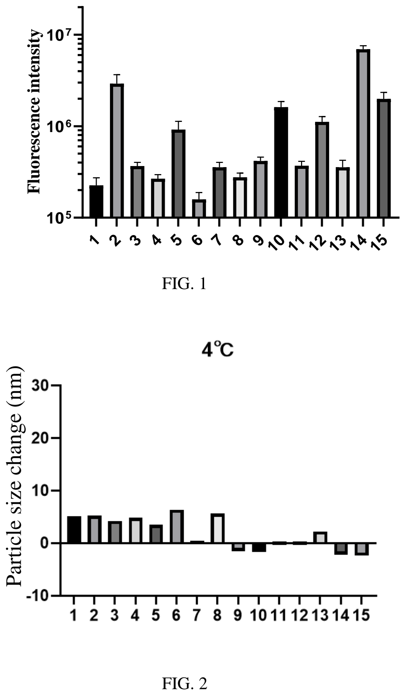

HEK 293 cells were seeded in a 96-well plate and cultured overnight. When the cell density reached 80% or higher, solutions of the lipid nanoparticles in which the luciferase mRNA was encapsulated were added to the culture medium in the wells of the cell plate at an mRNA dose of 100 ng/well. After 24 h, the fluorescence intensity of the expressed luciferase protein was measured using a luciferase reporter gene assay kit (Promega) and a microplate reader. The fluorescence intensity values, i.e., the fluorescence measurements obtained using the microplate reader, represent expression levels of luciferase protein. The higher the fluorescence intensity, the higher the protein expression level. For the lipid nanoparticles corresponding to each compound, at least 3 repetitions were performed, and the mean fluorescence intensity was calculated. The data are shown in Table 2 and FIG. 1.

**Table 2**

| No. | Mean fluorescence intensity |
|---|---|
| 1 | 2.26E+05 |
| 2 | 2.92E+06 |
| 3 | 3.67E+05 |
| 4 | 2.67E+05 |
| 5 | 9.21E+05 |
| 6 | 1.59E+05 |
| 7 | 3.56E+05 |
| 8 | 2.77E+05 |
| 9 | 4.17E+05 |
| 10 | 1.62E+06 |
| 11 | 3.71E+05 |
| 12 | 1.12E+06 |
| 13 | 3.55E+05 |
| 14 | 6.95E+06 |
| 15 | 1.99E+06 |

### Test Example 2: Evaluation of Stability of Lipid Particle Compositions

The lipid nanoparticles corresponding to lipid compositions 1-15 were separately placed in a 4 °C environment and a 25 °C environment. After 3 days of standing, the particle size of the lipid nanoparticles in each group was measured, and the particle size changes during storage were compared to assess the stability of the lipid nanoparticles. A big particle size change indicates poor stability. Each group was repeatedly tested 3 times, and the average particle size was calculated, as shown in Table 3. The differences between the particle sizes at 4 °C and 25 °C and the initial particle size of the lipid nanoparticles were calculated. The smaller the deviations of the differences from 0, the smaller the particle size changes within the storage period, and the more stable the lipid nanoparticles. As shown in Table 3, after 3 days of standing in the 4 °C and 25 °C environments, the polydispersity index of the particle size was determined by dynamic light scattering. The particle size changes are shown in Table 4 and FIGs. 2-3.

**Table 3**

| No. | 4 °C | | 25 °C | |
|---|---|---|---|---|
| | Particle size (nm) | PDI | Particle size (nm) | PDI |
| 1 | 92.3 | 0.29 | 109.1 | 0.30 |
| 2 | 118.6 | 0.17 | 119.5 | 0.18 |
| 3 | 84.0 | 0.22 | 94.6 | 0.24 |
| 4 | 80.8 | 0.27 | 100.1 | 0.25 |
| 5 | 95.4 | 0.19 | 104.5 | 0.21 |
| 6 | 82.9 | 0.23 | 95.2 | 0.25 |
| 7 | 89.2 | 0.18 | 95.7 | 0.19 |
| 8 | 91.5 | 0.23 | 104.3 | 0.25 |
| 9 | 69.2 | 0.15 | 70.8 | 0.13 |
| 10 | 97.6 | 0.13 | 97.8 | 0.16 |
| 11 | 70.8 | 0.13 | 73.1 | 0.14 |
| 12 | 83.8 | 0.18 | 83.7 | 0.16 |
| 13 | 72.7 | 0.23 | 81.7 | 0.22 |
| 14 | 88.6 | 0.10 | 88.8 | 0.09 |
| 15 | 81.6 | 0.12 | 82.5 | 0.17 |

**Table 4**

| No. | 4 °C | 25 °C |
|---|---|---|
| | Particle size change (nm) | |
| 1 | 5.1 | 21.9 |
| 2 | 5.2 | 6.1 |
| 3 | 4.2 | 14.9 |
| 4 | 4.8 | 24.0 |
| 5 | 3.5 | 12.6 |
| 6 | 6.4 | 18.7 |
| 7 | -0.1 | 6.3 |
| 8 | 5.6 | 18.4 |
| 9 | -1.5 | 0.1 |
| 10 | -1.7 | -1.5 |
| 11 | 0.3 | 2.6 |
| 12 | -0.3 | -0.4 |
| 13 | 2.2 | 11.2 |
| 14 | -2.2 | -2.1 |
| 15 | -2.4 | -1.6 |

### Example 3: Preparation of mRNA Vaccines

In the present disclosure, we prepared a variety of mRNA products and illustratively presented VZV mRNA vaccines. Their preparation and verification examples are shown below.

### 3.1. Design and synthesis of VZV mRNA vaccine DNA templates

To improve the expression of the VZV gE protein and the stability of its structure in human cells, this example performed codon optimization and designed different C-terminal variant molecules encoding the gE protein of the Oka strain. The amino acid sequence of the gE protein of the Oka strain is set forth in SEQ ID NO: 1, and the nucleotide sequence is set forth in SEQ ID NO: 2. We designed 6 gE molecules: gE1 (Oka gE, 1-546aa), gE2 (Oka gE, 1-574aa & Y569A), gE3 (Oka gE, 1-544aa), gE4 (Oka gE, 1-574aa), gE5 (Oka gE, 1-561aa), and one full-length gE6 (Oka gE, 1-623aa). The amino acid sequences and coding nucleic acid sequences of the gE1-gE6 molecules are shown in Table 5. gEl-gE5 were C-terminally truncated gE molecules.

**Table 5. VZV mRNA vaccine sequences**

| gE molecule | Nucleic acid sequence | Amino acid sequence |
|---|---|---|
| gE1 | 3 | 21 |
| gE2 | 4 | 22 |
| gE3 | 5 | 23 |
| gE4 | 6 | 24 |
| gE5 | 7 | 25 |
| gE6 | 8 | 1 |

A T7 promoter sequence (TAATACGACTCACTATAAG), a 5'-UTR sequence (its corresponding RNA sequence is set forth in SEQ ID NO: 9), a 3'-UTR sequence (its corresponding RNA sequence is set forth in SEQ ID NO: 10), and a poly(A) sequence (its corresponding RNA sequence is set forth in SEQ ID NO: 11) were also designed.

Then, the T7 promoter sequence, the 5'-UTR sequence, the DNA ORF, the 3'-UTR sequence, and the poly(A) sequence were ligated in this order, and whole-gene synthesis was performed with pUC57 as a vector (Suzhou GENEWIZ Biotechnology Co., Ltd.) to obtain plasmid DNA templates.

### 3.2. Preparation of VZV mRNA vaccine mRNA stock solutions

The plasmid DNAs obtained in Example 3.1 were digested with XbaI and purified to obtain linearized plasmid DNAs. A co-transcriptional capping (Cap1 type) reaction was performed through T7 RNA polymerase, and transcription was performed *in vitro* to produce the desired RNAs. In the *in vitro* transcription, (1-methyl)-pseudouridine-triphosphate was used in place of uridine triphosphate (UTP). After the end of the transcription, the DNA templates were digested with DNase I to reduce the risk posed by DNA template residues. The mRNAs were purified using AKTA, and the purified mRNAs were dissolved in sterile water and cryopreserved at -80 °C until they were used.

### 3.3. Preparation of VZV mRNA vaccine mRNA-LNP formulations

Compound 1 (the compound of formula I), DSPC, cholesterol, and DMG-PEG 2000 were dissolved at a molar ratio of 48:10:40.5:1.5 in ethanol to prepare a lipid solution. Each of the mRNAs was dissolved in a 50 mM pH 4 acetate buffer to prepare an aqueous mRNA solution. The ethanol lipid solution and the aqueous mRNA solution were mixed by microfluidics at a volume ratio of 1:3 (the weight ratio of compound 1 to the mRNA in the lipid was 12:1) to prepare lipid nanoparticles. The ethanol was removed by dialysis against a 20 mM Tris pH 7.5 solution, and the lipid nanoparticles were finally exchanged into a 20 mM Tris 8% sucrose solution and cryopreserved to obtain lipid nanoparticles in which the mRNA was encapsulated. The lipid nanoparticles were then cryopreserved at - 80 °C until they were used.

Example 4: Expression of VZV mRNA Vaccines in Cells and Immunogenicity Thereof in Mice

### 4.1. Assay for in vitro expression activity of VZV mRNA vaccines

HEK 293T cells (human kidney epithelial cell line) were cultured in a Dulbecco modified Eagle medium (DMEM) supplemented with 10% FBS and 1% penicillin-streptomycin at 37 °C with 5% CO2. First, we verified the *in vitro* expression of 6 gE-LNP mRNAs designed with 30L70A as poly A in HEK293 cells. This example used liquid formulations of 6 gE-mRNA LNPs containing 30L70A and using (1-methyl)-pseudouridine-triphosphate in place of UTP as prepared in Example 3. The nucleotide sequences were gE1-LNP (SEQ ID NO: 13), gE2-LNP (SEQ ID NO: 14), gE3-LNP (SEQ ID NO: 15), gE4-LNP (SEQ ID NO: 16), gE5-LNP (SEQ ID NO: 17), and gE6-LNP (SEQ ID NO: 18).

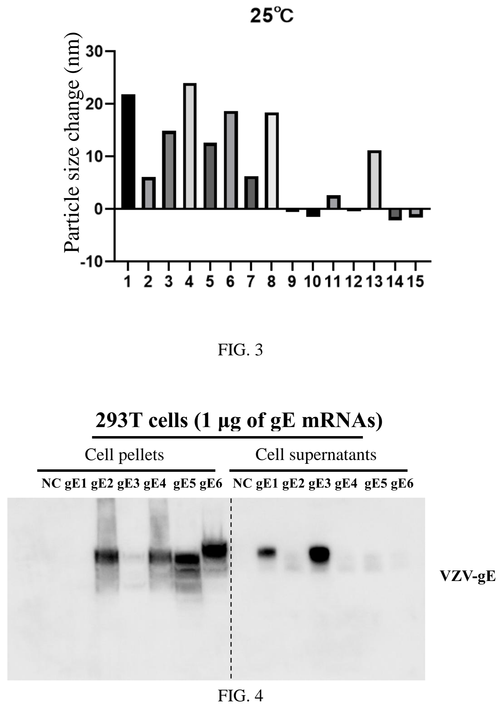

HEK-293T cells were seeded in a six-well plate at 1.2 × 10⁶ cells/well and incubated in an incubator at 37 °C for 18-24 h. Formulations of gE-mRNA LNPs containing 1 µg of mRNA were directly added to the HEK-293T cells, and the cells were incubated for another 24 h. The culture supernatants or cell pellets were collected and assayed by Western Blot. The mouse anti-VZV gE Ab used in the Western Blot assay was purchased from Abcam, and the Goat anti-mouse IgG HRP Ab was purchased from TransGen. The Western Blot results are shown in FIG. 4. The results show that: 1) the 6 gE-mRNA-LNPs all expressed the corresponding gE proteins; 2) among gE2, gE4, gE5, and gE6, which retained the C-terminal transmembrane region, gE2, gE5, and gE6 exhibited relatively high *in vitro* expression activity.

### 4.2. Immunization of C57 mice with VZV mRNA vaccines

This example used female C57 mice aged 6-8 weeks. The animal study was conducted in strict accordance with the recommendations in the Shanghai Guide for the Care and Use of Laboratory Animals.

This example used formulations of 6 gE-LNP mRNAs as prepared in Example 4.1 to immunize the C57 mice and evaluated the levels of gE-specific antibodies that they induced in the mice.

The mice were randomly divided into 7 groups (n = 7). Six of the groups were immunized with formulations of gE-LNP mRNAs as prepared in Example 3 (single doses were formulations of gE-mRNA LNPs containing 2 µg of mRNA), respectively, and the other group was given phosphate-buffered saline (PBS) as a negative control group. Each group was dosed by intramuscular injection on day 0 (prime immunization) and day 14 (secondary immunization). On day 14 after the secondary immunization of the mice (i.e., on day 28), blood was collected from the orbit, and mouse serum was collected and assayed for antibodies.

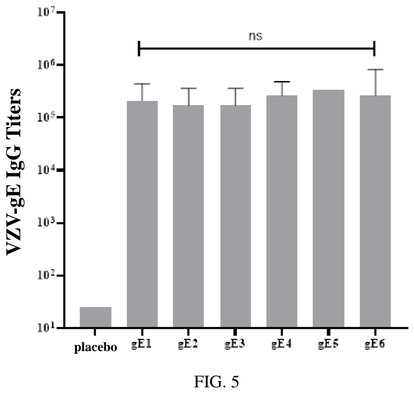

The serum anti-VZV gE IgG assay (gP ELISA) was as follows: VZV-gE antigen (ACROBiosystems) was diluted with ELISA coating Buffer to 0.25 µg/mL, and 100 µL of 0.25 µg/mL VZV-gE antigen was added to each well using a multi-channel pipette. The plates were incubated overnight at 4 °C. The test samples were equilibrated at room temperature for 30 min. The plates were washed with PBST (0.05% Tween-20) and blocked with 5% skim milk in PBST. The serum samples were diluted with 2.5% skim milk powder, with an initial dilution titer of 1:50. Then, the test serum samples were diluted 2-fold. A negative serum control was set in each plate. The diluted serum samples were added at 100 µL/well, and the plates were incubated at 37 °C (in a thermostatic incubator) for 1 h. The serum samples were discarded. 250 µL of PBST wash buffer was added to each well using a multi-channel pipette, and the plates were washed 3 times. 100 µL of 50,000-fold diluted goat anti-mouse IgG HRP (abcam) was added to each well, and the plates were incubated at 37 °C (in a thermostatic incubator) for 0.5 h. The secondary antibody was discarded. 300 µL of PBST wash buffer was added to each well using a multi-channel pipette, and the plates were washed 5 times. After washing, 100 µL of 3,3',5,5'-tetramethylbenzidine (TMB) (ebioscience) substrate solution was added to each well for color development (5 min, in a dark place). 100 µL of 2 N H2SO4 was added to each well to stop the reaction, and detection was performed. The OD values were read at a wavelength of 450 nm using a microplate reader (BioTek). The endpoint titer was determined to be the reciprocal of the serum of the last dilution. A sample with an OD450 nm greater than 2.1 times the OD450 nm of the negative control group was determined to be positive. The results are shown in FIG. 5. The results show that the 6 gE LNP-mRNA molecules induced comparable levels of humoral immunity.

Some of the sequences of the present disclosure are shown as follows:
> Amino acid sequence of gE protein of Oka strain 
MGTVNKPVVGVLMGFGIITGTLRITNPVRASVLRYDDFHIDEDKLDTNSVYEPY
YHSDHAESSWVNRGESSRKAYDHNSPYIWPRNDYDGFLENAHEHHGVYNQGR
GIDSGERLMQPTQMSAQEDLGDDTGIHVIPTLNGDDRHKIVNVDQRQYGDVFK
GDLNPKPQGQRLIEVSVEENHPFTLRAPIQRIYGVRYTETWSFLPSLTCTGDAAP
AIQHICLKHTTCFQDVVVDVDCAENTKEDQLAEISYRFQGKKEADQPWIVVNT
STLFDELELDPPEIEPGVLKVLRTEKQYLGVYIWNMRGSDGTSTYATFLVTWKG
DEKTRNPTPAVTPQPRGAEFHMWNYHSHVFSVGDTFSLAMHLQYKIHEAPFDL
LLEWLYVPIDPTCQPMRLYSTCLYHPNAPQCLSHMNSGCTFTSPHLAQRVASTV
YQNCEHADNYTAYCLGISHMEPSFGLILHDGGTTLKFVDTPESLSGLYVFVVYF
NGHVEAVAYTVVSTVDHFVNAIEERGFPPTAGQPPATTKPKEITPVNPGTSPLLRY
AAWTGGLAAVVLLCLVIFLICTAKRMRVKAYRVDKSPYNQSMYYAGLPVDDFE
DSESTDTEEEFGNAIGGSHGGSSYTVYIDKTR
SEQ ID NO: 1
> Nucleotide sequence of gE protein of Oka strain 
ATGGGGACAGTTAATAAACCTGTGGTGGGGGTATTGATGGGGTTCGGAATTAT
CACGGGAACGTTGCGTATAACGAATCCGGTCAGAGCATCCGTCTTGCGATAC
GATGATTTTCACATCGATGAAGACAAACTGGATACAAACTCCGTATATGAGCC
TTACTACCATTCAGATCATGCGGAGTCTTCATGGGTAAATCGGGGAGAGTCTT
CGCGAAAAGCGTACGATCATAACTCACCTTATATATGGCCACGTAATGATTATG
ATGGATTTTTAGAGAACGCACACGAACACCATGGGGTGTATAATCAGGGCCG
TGGTATCGATAGCGGGGAACGGTTAATGCAACCCACACAAATGTCTGCACAG
GAGGATCTTGGGGACGATACGGGCATCCACGTTATCCCTACGTTAAACGGCG
ATGACAGACATAAAATTGTAAATGTGGACCAACGTCAATACGGTGACGTGTT
TAAAGGAGATCTTAATCCAAAACCCCAAGGCCAAAGACTCATTGAGGTGTCA
GTGGAAGAAAATCACCCGTTTACTTTACGCGCACCGATTCAGCGGATTTATGG
AGTCCGGTACACCGAGACTTGGAGCTTTTTGCCGTCATTAACCTGTACGGGA
GACGCAGCGCCCGCCATCCAGCATATATGTTTAAAACATACAACATGCTTTCA
 
AGACGTGGTGGTGGATGTGGATTGCGCGGAAAATACTAAAGAGGATCAGTTG
GCCGAAATCAGTTACCGTTTTCAAGGTAAGAAGGAAGCGGACCAACCGTGG
ATTGTTGTAAACACGAGCACACTGTTTGATGAACTCGAATTAGACCCCCCCG
AGATTGAACCGGGTGTCTTGAAAGTACTTCGGACAGAAAAACAATACTTGGG
TGTGTACATTTGGAACATGCGCGGCTCCGATGGTACGTCTACCTACGCCACGT
TTTTGGTCACCTGGAAAGGGGATGAAAAAACAAGAAACCCTACGCCCGCAG
TAACTCCTCAACCAAGAGGGGCTGAGTTTCATATGTGGAATTACCACTCGCAT
GTATTTTCAGTTGGTGATACGTTTAGCTTGGCAATGCATCTTCAGTATAAGATA
CATGAAGCGCCATTTGATTTGCTGTTAGAGTGGTTGTATGTCCCCATCGATCCT
ACATGTCAACCAATGCGGTTATATTCTACGTGTTTGTATCATCCCAACGCACCC
CAATGCCTCTCTCATATGAATTCCGGTTGTACATTTACCTCGCCACATTTAGCC
CAGCGTGTTGCAAGCACAGTGTATCAAAATTGTGAACATGCAGATAACTACA
CCGCATATTGTCTGGGAATATCTCATATGGAGCCTAGCTTTGGTCTAATCTTAC
ACGACGGGGGCACCACGTTAAAGTTTGTAGATACACCCGAGAGTTTGTCGGG
ATTATACGTTTTTGTGGTGTATTTTAACGGGCATGTTGAAGCCGTAGCATACAC
TGTTGTATCCACAGTAGATCATTTTGTAAACGCAATTGAAGAGCGTGGATTTC
CGCCAACGGCCGGTCAGCCACCGGCGACTACTAAACCCAAGGAAATTACCC
CCGTAAACCCCGGAACGTCACCACTTCTACGATATGCCGCATGGACCGGAGG
GCTTGCAGCAGTAGTACTTTTATGTCTCGTAATATTTTTAATCTGTACGGCTAA
ACGAATGAGGGTTAAAGCCTATAGGGTAGACAAGTCCCCGTATAACCAAAGC
ATGTATTACGCTGGCCTTCCAGTGGACGATTTCGAGGACTCGGAATCTACGGA
TACGGAAGAAGAGTTTGGTAACGCGATTGGAGGGAGTCACGGGGGTTCGAG
TTACACGGTGTATATAGATAAGACCCGGTGA
SEQ ID NO: 2
> Nucleotide sequence of gE1 
AUGGGCACCGUGAACAAGCCCGUGGUGGGCGUGCUGAUGGGCUUCGGCA
UCAUCACCGGCACCCUGCGCAUCACCAACCCCGUGCGGGCCAGCGUGCUG
CGCUACGACGACUUCCACAUCGACGAGGACAAGCUGGACACCAACUCCGU
GUACGAGCCCUACUACCACAGCGACCACGCCGAGUCCAGCUGGGUGAACC
GGGGCGAGUCCAGCCGCAAGGCCUACGACCACAACUCCCCCUACAUCUGG
CCCCGGAACGACUACGACGGCUUCCUGGAGAACGCCCACGAGCACCACGG
GGUGUACAACCAGGGCCGCGGGAUCGACAGCGGCGAGCGGCUGAUGCAGC
CCACCCAGAUGUCCGCCCAGGAGGACCUGGGGGACGACACCGGCAUCCAC
GUGAUCCCCACCCUGAACGGGGACGACCGCCACAAGAUCGUGAACGUGGA
CCAGCGGCAGUACGGCGACGUGUUCAAGGGGGACCUGAACCCCAAGCCCC
AGGGCCAGCGCCUGAUCGAGGUCAGCGUGGAGGAGAACCACCCCUUCACC
CUGCGGGCCCCCAUCCAGCGCAUCUACGGGGUCCGGUACACCGAGACCUG
GUCCUUCCUGCCCAGCCUGACCUGCACCGGCGACGCCGCCCCCGCCAUCCA
GCACAUCUGCCUGAAGCACACCACCUGCUUCCAGGACGUGGUCGUGGACG
UCGACUGCGCCGAGAACACCAAGGAGGACCAGCUCGCCGAGAUCUCCUAC
 
CGCUUCCAGGGGAAGAAGGAGGCCGACCAGCCCUGGAUCGUGGUCAACAC
CAGCACCCUGUUCGACGAGCUGGAGCUGGACCCGCCCGAGAUCGAGCCCG
GCGUGCUCAAGGUCCUGCGGACCGAGAAGCAGUACCUCGGGGUGUACAUC
UGGAACAUGCGCGGCUCCGACGGGACCAGCACCUACGCCACCUUCCUGGU
CACCUGGAAGGGCGACGAGAAGACCCGGAACCCCACCCCCGCCGUGACGC
CCCAGCCGCGCGGGGCCGAGUUCCACAUGUGGAACUACCACUCCCACGUC
UUCAGCGUGGGCGACACGUUCUCCCUCGCCAUGCACCUGCAGUACAAGAU
CCACGAGGCCCCGUUCGACCUCCUGCUGGAGUGGCUGUACGUCCCCAUCG
ACCCGACCUGCCAGCCCAUGCGGCUCUACAGCACGUGCCUGUACCACCCC
AACGCCCCGCAGUGCCUCUCCCACAUGAACAGCGGGUGCACCUUCACGUC
CCCGCACCUGGCGCAGCGCGUGGCCAGCACCGUCUACCAGAACUGCGAGC
ACGCGGACAACUACACCGCCUACUGCCUCGGCAUCUCCCACAUGGAGCCC
UCGUUCGGGCUGAUCCUCCACGACGGCGGGACGACCCUGAAGUUCGUGGA
CACGCCGGAGAGCCUCUCCGGCCUGUACGUCUUCGUGGUCUACUUCAACG
GGCACGUGGAGGCGGUCGCCUACACGGUGGUCUCGACCGUGGACCACUUC
GUCAACGCGAUCGAGGAGCGGGGCUUUCCCCCGACCGCCGGGCAGCCGCC
CGCGACGACGAAGCCGAAGGAGAUCACCCCGGUGAACCCCGGCACGAGCC
CGCUCCUGCGCUACGCCGCGUGGACCGGGGGCCUCGCCUGA
SEQ ID NO: 3
> Nucleotide sequence of gE2 
AUGGGCACCGUGAACAAGCCCGUGGUGGGCGUGCUGAUGGGCUUCGGCA
UCAUCACCGGCACCCUGCGCAUCACCAACCCCGUGCGGGCCAGCGUGCUG
CGCUACGACGACUUCCACAUCGACGAGGACAAGCUGGACACCAACUCCGU
GUACGAGCCCUACUACCACAGCGACCACGCCGAGUCCAGCUGGGUGAACC
GGGGCGAGUCCAGCCGCAAGGCCUACGACCACAACUCCCCCUACAUCUGG
CCCCGGAACGACUACGACGGCUUCCUGGAGAACGCCCACGAGCACCACGG
GGUGUACAACCAGGGCCGCGGGAUCGACAGCGGCGAGCGGCUGAUGCAGC
CCACCCAGAUGUCCGCCCAGGAGGACCUGGGGGACGACACCGGCAUCCAC
GUGAUCCCCACCCUGAACGGGGACGACCGCCACAAGAUCGUGAACGUGGA
CCAGCGGCAGUACGGCGACGUGUUCAAGGGGGACCUGAACCCCAAGCCCC
AGGGCCAGCGCCUGAUCGAGGUCAGCGUGGAGGAGAACCACCCCUUCACC
CUGCGGGCCCCCAUCCAGCGCAUCUACGGGGUCCGGUACACCGAGACCUG
GUCCUUCCUGCCCAGCCUGACCUGCACCGGCGACGCCGCCCCCGCCAUCCA
GCACAUCUGCCUGAAGCACACCACCUGCUUCCAGGACGUGGUCGUGGACG
UCGACUGCGCCGAGAACACCAAGGAGGACCAGCUCGCCGAGAUCUCCUAC
CGCUUCCAGGGGAAGAAGGAGGCCGACCAGCCCUGGAUCGUGGUCAACAC
CAGCACCCUGUUCGACGAGCUGGAGCUGGACCCGCCCGAGAUCGAGCCCG
GCGUGCUCAAGGUCCUGCGGACCGAGAAGCAGUACCUCGGGGUGUACAUC
UGGAACAUGCGCGGCUCCGACGGGACCAGCACCUACGCCACCUUCCUGGU
CACCUGGAAGGGCGACGAGAAGACCCGGAACCCCACCCCCGCCGUGACGC
 
CCCAGCCGCGCGGGGCCGAGUUCCACAUGUGGAACUACCACUCCCACGUC
UUCAGCGUGGGCGACACGUUCUCCCUCGCCAUGCACCUGCAGUACAAGAU
CCACGAGGCCCCGUUCGACCUCCUGCUGGAGUGGCUGUACGUCCCCAUCG
ACCCGACCUGCCAGCCCAUGCGGCUCUACAGCACGUGCCUGUACCACCCC
AACGCCCCGCAGUGCCUCUCCCACAUGAACAGCGGGUGCACCUUCACGUC
CCCGCACCUGGCGCAGCGCGUGGCCAGCACCGUCUACCAGAACUGCGAGC
ACGCGGACAACUACACCGCCUACUGCCUCGGCAUCUCCCACAUGGAGCCC
UCGUUCGGGCUGAUCCUCCACGACGGCGGGACGACCCUGAAGUUCGUGGA
CACGCCGGAGAGCCUCUCCGGCCUGUACGUCUUCGUGGUCUACUUCAACG
GGCACGUGGAGGCGGUCGCCUACACGGUGGUCUCGACCGUGGACCACUUC
GUCAACGCGAUCGAGGAGCGGGGCUUUCCCCCGACCGCCGGGCAGCCGCC
CGCGACGACGAAGCCGAAGGAGAUCACCCCGGUGAACCCCGGCACGAGCC
CGCUCCUGCGCUACGCCGCGUGGACCGGGGGCCUCGCCGCGGUCGUGCUG
CUCUGCCUGGUCAUCUUCCUCAUCUGCACCGCCAAGCGGAUGCGCGUGAA
GGCGGCCCGGGUCGACAAGUCCUGA
SEQ ID NO: 4
> Nucleotide sequence of gE3 
AUGGGCACCGUGAACAAGCCCGUGGUGGGCGUGCUGAUGGGCUUCGGCA
UCAUCACCGGCACCCUGCGCAUCACCAACCCCGUGCGGGCCAGCGUGCUG
CGCUACGACGACUUCCACAUCGACGAGGACAAGCUGGACACCAACUCCGU
GUACGAGCCCUACUACCACAGCGACCACGCCGAGUCCAGCUGGGUGAACC
GGGGCGAGUCCAGCCGCAAGGCCUACGACCACAACUCCCCCUACAUCUGG
CCCCGGAACGACUACGACGGCUUCCUGGAGAACGCCCACGAGCACCACGG
GGUGUACAACCAGGGCCGCGGGAUCGACAGCGGCGAGCGGCUGAUGCAGC
CCACCCAGAUGUCCGCCCAGGAGGACCUGGGGGACGACACCGGCAUCCAC
GUGAUCCCCACCCUGAACGGGGACGACCGCCACAAGAUCGUGAACGUGGA
CCAGCGGCAGUACGGCGACGUGUUCAAGGGGGACCUGAACCCCAAGCCCC
AGGGCCAGCGCCUGAUCGAGGUCAGCGUGGAGGAGAACCACCCCUUCACC
CUGCGGGCCCCCAUCCAGCGCAUCUACGGGGUCCGGUACACCGAGACCUG
GUCCUUCCUGCCCAGCCUGACCUGCACCGGCGACGCCGCCCCCGCCAUCCA
GCACAUCUGCCUGAAGCACACCACCUGCUUCCAGGACGUGGUCGUGGACG
UCGACUGCGCCGAGAACACCAAGGAGGACCAGCUCGCCGAGAUCUCCUAC
CGCUUCCAGGGGAAGAAGGAGGCCGACCAGCCCUGGAUCGUGGUCAACAC
CAGCACCCUGUUCGACGAGCUGGAGCUGGACCCGCCCGAGAUCGAGCCCG
GCGUGCUCAAGGUCCUGCGGACCGAGAAGCAGUACCUCGGGGUGUACAUC
UGGAACAUGCGCGGCUCCGACGGGACCAGCACCUACGCCACCUUCCUGGU
CACCUGGAAGGGCGACGAGAAGACCCGGAACCCCACCCCCGCCGUGACGC
CCCAGCCGCGCGGGGCCGAGUUCCACAUGUGGAACUACCACUCCCACGUC
UUCAGCGUGGGCGACACGUUCUCCCUCGCCAUGCACCUGCAGUACAAGAU
CCACGAGGCCCCGUUCGACCUCCUGCUGGAGUGGCUGUACGUCCCCAUCG
ACCCGACCUGCCAGCCCAUGCGGCUCUACAGCACGUGCCUGUACCACCCC
 
AACGCCCCGCAGUGCCUCUCCCACAUGAACAGCGGGUGCACCUUCACGUC
CCCGCACCUGGCGCAGCGCGUGGCCAGCACCGUCUACCAGAACUGCGAGC
ACGCGGACAACUACACCGCCUACUGCCUCGGCAUCUCCCACAUGGAGCCC
UCGUUCGGGCUGAUCCUCCACGACGGCGGGACGACCCUGAAGUUCGUGGA
CACGCCGGAGAGCCUCUCCGGCCUGUACGUCUUCGUGGUCUACUUCAACG
GGCACGUGGAGGCGGUCGCCUACACGGUGGUCUCGACCGUGGACCACUUC
GUCAACGCGAUCGAGGAGCGGGGCUUUCCCCCGACCGCCGGGCAGCCGCC
CGCGACGACGAAGCCGAAGGAGAUCACCCCGGUGAACCCCGGCACGAGCC
CGCUCCUGCGCUACGCCGCGUGGACCGGGGGCUGA
SEQ ID NO: 5
> Nucleotide sequence of gE4 
AUGGGCACCGUGAACAAGCCCGUGGUGGGCGUGCUGAUGGGCUUCGGCA
UCAUCACCGGCACCCUGCGCAUCACCAACCCCGUGCGGGCCAGCGUGCUG
CGCUACGACGACUUCCACAUCGACGAGGACAAGCUGGACACCAACUCCGU
GUACGAGCCCUACUACCACAGCGACCACGCCGAGUCCAGCUGGGUGAACC
GGGGCGAGUCCAGCCGCAAGGCCUACGACCACAACUCCCCCUACAUCUGG
CCCCGGAACGACUACGACGGCUUCCUGGAGAACGCCCACGAGCACCACGG
GGUGUACAACCAGGGCCGCGGGAUCGACAGCGGCGAGCGGCUGAUGCAGC
CCACCCAGAUGUCCGCCCAGGAGGACCUGGGGGACGACACCGGCAUCCAC
GUGAUCCCCACCCUGAACGGGGACGACCGCCACAAGAUCGUGAACGUGGA
CCAGCGGCAGUACGGCGACGUGUUCAAGGGGGACCUGAACCCCAAGCCCC
AGGGCCAGCGCCUGAUCGAGGUCAGCGUGGAGGAGAACCACCCCUUCACC
CUGCGGGCCCCCAUCCAGCGCAUCUACGGGGUCCGGUACACCGAGACCUG
GUCCUUCCUGCCCAGCCUGACCUGCACCGGCGACGCCGCCCCCGCCAUCCA
GCACAUCUGCCUGAAGCACACCACCUGCUUCCAGGACGUGGUCGUGGACG
UCGACUGCGCCGAGAACACCAAGGAGGACCAGCUCGCCGAGAUCUCCUAC
CGCUUCCAGGGGAAGAAGGAGGCCGACCAGCCCUGGAUCGUGGUCAACAC
CAGCACCCUGUUCGACGAGCUGGAGCUGGACCCGCCCGAGAUCGAGCCCG
GCGUGCUCAAGGUCCUGCGGACCGAGAAGCAGUACCUCGGGGUGUACAUC
UGGAACAUGCGCGGCUCCGACGGGACCAGCACCUACGCCACCUUCCUGGU
CACCUGGAAGGGCGACGAGAAGACCCGGAACCCCACCCCCGCCGUGACGC
CCCAGCCGCGCGGGGCCGAGUUCCACAUGUGGAACUACCACUCCCACGUC
UUCAGCGUGGGCGACACGUUCUCCCUCGCCAUGCACCUGCAGUACAAGAU
CCACGAGGCCCCGUUCGACCUCCUGCUGGAGUGGCUGUACGUCCCCAUCG
ACCCGACCUGCCAGCCCAUGCGGCUCUACAGCACGUGCCUGUACCACCCC
AACGCCCCGCAGUGCCUCUCCCACAUGAACAGCGGGUGCACCUUCACGUC
CCCGCACCUGGCGCAGCGCGUGGCCAGCACCGUCUACCAGAACUGCGAGC
ACGCGGACAACUACACCGCCUACUGCCUCGGCAUCUCCCACAUGGAGCCC
UCGUUCGGGCUGAUCCUCCACGACGGCGGGACGACCCUGAAGUUCGUGGA
CACGCCGGAGAGCCUCUCCGGCCUGUACGUCUUCGUGGUCUACUUCAACG
GGCACGUGGAGGCGGUCGCCUACACGGUGGUCUCGACCGUGGACCACUUC
 
GUCAACGCGAUCGAGGAGCGGGGCUUUCCCCCGACCGCCGGGCAGCCGCC
CGCGACGACGAAGCCGAAGGAGAUCACCCCGGUGAACCCCGGCACGAGCC
CGCUCCUGCGCUACGCCGCGUGGACCGGGGGCCUCGCCGCGGUCGUGCUG
CUCUGCCUGGUCAUCUUCCUCAUCUGCACCGCCAAGCGGAUGCGCGUGAA
GGCGUACCGGGUCGACAAGUCCUGA
SEQ ID NO: 6
> Nucleotide sequence of gE5 
AUGGGCACCGUGAACAAGCCCGUGGUGGGCGUGCUGAUGGGCUUCGGCA
UCAUCACCGGCACCCUGCGCAUCACCAACCCCGUGCGGGCCAGCGUGCUG
CGCUACGACGACUUCCACAUCGACGAGGACAAGCUGGACACCAACUCCGU
GUACGAGCCCUACUACCACAGCGACCACGCCGAGUCCAGCUGGGUGAACC
GGGGCGAGUCCAGCCGCAAGGCCUACGACCACAACUCCCCCUACAUCUGG
CCCCGGAACGACUACGACGGCUUCCUGGAGAACGCCCACGAGCACCACGG
GGUGUACAACCAGGGCCGCGGGAUCGACAGCGGCGAGCGGCUGAUGCAGC
CCACCCAGAUGUCCGCCCAGGAGGACCUGGGGGACGACACCGGCAUCCAC
GUGAUCCCCACCCUGAACGGGGACGACCGCCACAAGAUCGUGAACGUGGA
CCAGCGGCAGUACGGCGACGUGUUCAAGGGGGACCUGAACCCCAAGCCCC
AGGGCCAGCGCCUGAUCGAGGUCAGCGUGGAGGAGAACCACCCCUUCACC
CUGCGGGCCCCCAUCCAGCGCAUCUACGGGGUCCGGUACACCGAGACCUG
GUCCUUCCUGCCCAGCCUGACCUGCACCGGCGACGCCGCCCCCGCCAUCCA
GCACAUCUGCCUGAAGCACACCACCUGCUUCCAGGACGUGGUCGUGGACG
UCGACUGCGCCGAGAACACCAAGGAGGACCAGCUCGCCGAGAUCUCCUAC
CGCUUCCAGGGGAAGAAGGAGGCCGACCAGCCCUGGAUCGUGGUCAACAC
CAGCACCCUGUUCGACGAGCUGGAGCUGGACCCGCCCGAGAUCGAGCCCG
GCGUGCUCAAGGUCCUGCGGACCGAGAAGCAGUACCUCGGGGUGUACAUC
UGGAACAUGCGCGGCUCCGACGGGACCAGCACCUACGCCACCUUCCUGGU
CACCUGGAAGGGCGACGAGAAGACCCGGAACCCCACCCCCGCCGUGACGC
CCCAGCCGCGCGGGGCCGAGUUCCACAUGUGGAACUACCACUCCCACGUC
UUCAGCGUGGGCGACACGUUCUCCCUCGCCAUGCACCUGCAGUACAAGAU
CCACGAGGCCCCGUUCGACCUCCUGCUGGAGUGGCUGUACGUCCCCAUCG
ACCCGACCUGCCAGCCCAUGCGGCUCUACAGCACGUGCCUGUACCACCCC
AACGCCCCGCAGUGCCUCUCCCACAUGAACAGCGGGUGCACCUUCACGUC
CCCGCACCUGGCGCAGCGCGUGGCCAGCACCGUCUACCAGAACUGCGAGC
ACGCGGACAACUACACCGCCUACUGCCUCGGCAUCUCCCACAUGGAGCCC
UCGUUCGGGCUGAUCCUCCACGACGGCGGGACGACCCUGAAGUUCGUGGA
CACGCCGGAGAGCCUCUCCGGCCUGUACGUCUUCGUGGUCUACUUCAACG
GGCACGUGGAGGCGGUCGCCUACACGGUGGUCUCGACCGUGGACCACUUC
GUCAACGCGAUCGAGGAGCGGGGCUUUCCCCCGACCGCCGGGCAGCCGCC
CGCGACGACGAAGCCGAAGGAGAUCACCCCGGUGAACCCCGGCACGAGCC
CGCUCCUGCGCUACGCCGCGUGGACCGGGGGCCUCGCCGCGGUCGUGCUG
CUCUGCCUGGUCAUCUUCCUCAUCUGCACCGCCUGA
 SEQ ID NO: 7
> Nucleotide sequence of gE6 
AUGGGCACCGUGAACAAGCCCGUGGUGGGCGUGCUGAUGGGCUUCGGCA
UCAUCACCGGCACCCUGCGCAUCACCAACCCCGUGCGGGCCAGCGUGCUG
CGCUACGACGACUUCCACAUCGACGAGGACAAGCUGGACACCAACUCCGU
GUACGAGCCCUACUACCACAGCGACCACGCCGAGUCCAGCUGGGUGAACC
GGGGCGAGUCCAGCCGCAAGGCCUACGACCACAACUCCCCCUACAUCUGG
CCCCGGAACGACUACGACGGCUUCCUGGAGAACGCCCACGAGCACCACGG
GGUGUACAACCAGGGCCGCGGGAUCGACAGCGGCGAGCGGCUGAUGCAGC
CCACCCAGAUGUCCGCCCAGGAGGACCUGGGGGACGACACCGGCAUCCAC
GUGAUCCCCACCCUGAACGGGGACGACCGCCACAAGAUCGUGAACGUGGA
CCAGCGGCAGUACGGCGACGUGUUCAAGGGGGACCUGAACCCCAAGCCCC
AGGGCCAGCGCCUGAUCGAGGUCAGCGUGGAGGAGAACCACCCCUUCACC
CUGCGGGCCCCCAUCCAGCGCAUCUACGGGGUCCGGUACACCGAGACCUG
GUCCUUCCUGCCCAGCCUGACCUGCACCGGCGACGCCGCCCCCGCCAUCCA
GCACAUCUGCCUGAAGCACACCACCUGCUUCCAGGACGUGGUCGUGGACG
UCGACUGCGCCGAGAACACCAAGGAGGACCAGCUCGCCGAGAUCUCCUAC
CGCUUCCAGGGGAAGAAGGAGGCCGACCAGCCCUGGAUCGUGGUCAACAC
CAGCACCCUGUUCGACGAGCUGGAGCUGGACCCGCCCGAGAUCGAGCCCG
GCGUGCUCAAGGUCCUGCGGACCGAGAAGCAGUACCUCGGGGUGUACAUC
UGGAACAUGCGCGGCUCCGACGGGACCAGCACCUACGCCACCUUCCUGGU
CACCUGGAAGGGCGACGAGAAGACCCGGAACCCCACCCCCGCCGUGACGC
CCCAGCCGCGCGGGGCCGAGUUCCACAUGUGGAACUACCACUCCCACGUC
UUCAGCGUGGGCGACACGUUCUCCCUCGCCAUGCACCUGCAGUACAAGAU
CCACGAGGCCCCGUUCGACCUCCUGCUGGAGUGGCUGUACGUCCCCAUCG
ACCCGACCUGCCAGCCCAUGCGGCUCUACAGCACGUGCCUGUACCACCCC
AACGCCCCGCAGUGCCUCUCCCACAUGAACAGCGGGUGCACCUUCACGUC
CCCGCACCUGGCGCAGCGCGUGGCCAGCACCGUCUACCAGAACUGCGAGC
ACGCGGACAACUACACCGCCUACUGCCUCGGCAUCUCCCACAUGGAGCCC
UCGUUCGGGCUGAUCCUCCACGACGGCGGGACGACCCUGAAGUUCGUGGA
CACGCCGGAGAGCCUCUCCGGCCUGUACGUCUUCGUGGUCUACUUCAACG
GGCACGUGGAGGCGGUCGCCUACACGGUGGUCUCGACCGUGGACCACUUC
GUCAACGCGAUCGAGGAGCGGGGCUUUCCCCCGACCGCCGGGCAGCCGCC
CGCGACGACGAAGCCGAAGGAGAUCACCCCGGUGAACCCCGGCACGAGCC
CGCUCCUGCGCUACGCCGCGUGGACCGGGGGCCUCGCCGCGGUCGUGCUG
CUCUGCCUGGUCAUCUUCCUCAUCUGCACCGCCAAGCGGAUGCGCGUGAA
GGCGUACCGGGUCGACAAGUCCCCCUACAACCAGUCGAUGUACUACGCCG
GGCUGCCCGUGGACGACUUCGAGGACAGCGAGUCCACGGACACCGAGGAG
GAGUUCGGCAACGCGAUCGGGGGCUCGCACGGGGGCUCCUCGUACACGGU
CUACAUCGACAAGACCCGCUGA
SEQ ID NO: 8
> 5'UTR 
GAGUUAGGAGUUGGUGGCAGUUUCAAUAACAGGUCAUUGCCGAGAAAAG
GAUAGCACUAUAAU
SEQ ID NO: 9
> 3'UTR 
GCUCGCUUUCUUGCUGUCCAAUUUCUAUUAAAGGUUCCUUUGUUCCCUAA
GUCCAACUACUAAACUGGGGGAUAUUAUGAAGGGCCUUGAGCAUCUGGA
UUCUGCCUAAUAAAAAACAUUUAUUUUCAUUGCAA
SEQ ID NO: 10
> 30L70A polyA 
AAAAAAAAAAAAAAAAAAAAAAAAAAAAAAGCAUAUGACUAAAAAAAA
AAAAAAAAAAAAAAAAAAAAAAAAAAAAAAAAAAAAAAAAAAAAAAAA
AAAAAAAAAAAA
SEQ ID NO: 11
> 120A polyA 
AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
AAAAAAAAAAAAAAAAAAAAAA
SEQ ID NO: 12
> gE1 (30L70A) mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
GGAUCGUGGUCAACACCAGCACCCUGUUCGACGAGCUGGAGCUGGACCCG
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
ACGCCACCUUCCUGGUCACCUGGAAGGGCGACGAGAAGACCCGGAACCCC
 
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCCU
CGCCUGAUGAACCGGUGCUCGCUUUCUUGCUGUCCAAUUUCUAUUAAAGG
UUCCUUUGUUCCCUAAGUCCAACUACUAAACUGGGGGAUAUUAUGAAGG
GCCUUGAGCAUCUGGAUUCUGCCUAAUAAAAAACAUUUAUUUUCAUUGC
AAAAAAAAAAAAAAAAAAAAAAAAAAAAAAAAGCAUAUGACUAAAAAAA
AAAAAAAAAAAAAAAAAAAAAAAAAAAAAAAAAAAAAAAAAAAAAAAAAA
AAAAAAAAAAAAAAA
SEQ ID NO: 13
> gE2 (30L70A) mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
GGAUCGUGGUCAACACCAGCACCCUGUUCGACGAGCUGGAGCUGGACCCG
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
 
ACGCCACCUUCCUGGUCACCUGGAAGGGCGACGAGAAGACCCGGAACCCC
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCCU
CGCCGCGGUCGUGCUGCUCUGCCUGGUCAUCUUCCUCAUCUGCACCGCCA
AGCGGAUGCGCGUGAAGGCGGCCCGGGUCGACAAGUCCUGAUGAACCGGU
GCUCGCUUUCUUGCUGUCCAAUUUCUAUUAAAGGUUCCUUUGUUCCCUAA
GUCCAACUACUAAACUGGGGGAUAUUAUGAAGGGCCUUGAGCAUCUGGA
UUCUGCCUAAUAAAAAACAUUUAUUUUCAUUGCAAAAAAAAAAAAAAA
AAAAAAAAAAAAAAAAAGCAUAUGACUAAAAAAAAAAAAAAAAAAAAAAA
AAAAAAAAAAAAAAAAAAAAAAAAAAAAAAAAAAAAAAAAAAAAAA
SEQ ID NO: 14
> gE3 (30L70A) mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
GGAUCGUGGUCAACACCAGCACCCUGUUCGACGAGCUGGAGCUGGACCCG
 
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
ACGCCACCUUCCUGGUCACCUGGAAGGGCGACGAGAAGACCCGGAACCCC
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCUG
AUGAACCGGUGCUCGCUUUCUUGCUGUCCAAUUUCUAUUAAAGGUUCCU
UUGUUCCCUAAGUCCAACUACUAAACUGGGGGAUAUUAUGAAGGGCCUU
GAGCAUCUGGAUUCUGCCUAAUAAAAAACAUUUAUUUUCAUUGCAAAAA
AAAAAAAAAAAAAAAAAAAAAAAAAAAGCAUAUGACUAAAAAAAAAAAAA
AAAAAAAAAAAAAAAAAAAAAAAAAAAAAAAAAAAAAAAAAAAAAAAAAA
AAAAAAAAA
SEQ ID NO: 15
> gE4 (30L70A) mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
 
GGAUCGUGGUCAACACCAGCACCCUGUUCGACGAGCUGGAGCUGGACCCG
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
ACGCCACCUUCCUGGUCACCUGGAAGGGCGACGAGAAGACCCGGAACCCC
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCCU
CGCCGCGGUCGUGCUGCUCUGCCUGGUCAUCUUCCUCAUCUGCACCGCCA
AGCGGAUGCGCGUGAAGGCGUACCGGGUCGACAAGUCCUGAUGAACCGG
UGCUCGCUUUCUUGCUGUCCAAUUUCUAUUAAAGGUUCCUUUGUUCCCUA
AGUCCAACUACUAAACUGGGGGAUAUUAUGAAGGGCCUUGAGCAUCUGG
AUUCUGCCUAAUAAAAAACAUUUAUUUUCAUUGCAAAAAAAAAAAAAAA
AAAAAAAAAAAAAAAAAGCAUAUGACUAAAAAAAAAAAAAAAAAAAAAA
AAAAAAAAAAAAAAAAAAAAAAAAAAAAAAAAAAAAAAAAAAAAAAAAAA
SEQ ID NO: 16
> gE5 (30L70A) mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
 
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
GGAUCGUGGUCAACACCAGCACCCUGUUCGACGAGCUGGAGCUGGACCCG
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
ACGCCACCUUCCUGGUCACCUGGAAGGGCGACGAGAAGACCCGGAACCCC
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCCU
CGCCGCGGUCGUGCUGCUCUGCCUGGUCAUCUUCCUCAUCUGCACCGCCU
GAUGAACCGGUGCUCGCUUUCUUGCUGUCCAAUUUCUAUUAAAGGUUCC
UUUGUUCCCUAAGUCCAACUACUAAACUGGGGGAUAUUAUGAAGGGCCU
UGAGCAUCUGGAUUCUGCCUAAUAAAAAACAUUUAUUUUCAUUGCAAAA
AAAAAAAAAAAAAAAAAAAAAAAAAAAAGCAUAUGACUAAAAAAAAAAA
AAAAAAAAAAAAAAAAAAAAAAAAAAAAAAAAAAAAAAAAAAAAAAAAAA
AAAAAAAAAA
SEQ ID NO: 17
> gE6 (30L70A) mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
 
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
GGAUCGUGGUCAACACCAGCACCCUGUUCGACGAGCUGGAGCUGGACCCG
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
ACGCCACCUUCCUGGUCACCUGGAAGGGCGACGAGAAGACCCGGAACCCC
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCCU
CGCCGCGGUCGUGCUGCUCUGCCUGGUCAUCUUCCUCAUCUGCACCGCCA
AGCGGAUGCGCGUGAAGGCGUACCGGGUCGACAAGUCCCCCUACAACCAG
UCGAUGUACUACGCCGGGCUGCCCGUGGACGACUUCGAGGACAGCGAGUC
CACGGACACCGAGGAGGAGUUCGGCAACGCGAUCGGGGGCUCGCACGGGG
GCUCCUCGUACACGGUCUACAUCGACAAGACCCGCUGAUGAACCGGUGCU
CGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUC
UGCCUAAUAAAAAACAUUUAUUUUCAUUGCAAAAAAAAAAAAAAAAAAA
AAAAAAAAAAAAAGCAUAUGACUAAAAAAAAAAAAAAAAAAAAAAAAAA
AAAAAAAAAAAAAAAAAAAAAAAAAAAAAAAAAAAAAAAAAAAAAAAA
SEQ ID NO: 18
> gE2 120A mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
 
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
GGAUCGUGGUCAACACCAGCACCCUGUUCGACGAGCUGGAGCUGGACCCG
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
ACGCCACCUUCCUGGUCACCUGGAAGGGCGACGAGAAGACCCGGAACCCC
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCCU
CGCCGCGGUCGUGCUGCUCUGCCUGGUCAUCUUCCUCAUCUGCACCGCCA
AGCGGAUGCGCGUGAAGGCGGCCCGGGUCGACAAGUCCUGAUGAACCGGU
GCUCGCUUUCUUGCUGUCCAAUUUCUAUUAAAGGUUCCUUUGUUCCCUAA
GUCCAACUACUAAACUGGGGGAUAUUAUGAAGGGCCUUGAGCAUCUGGA
UUCUGCCUAAUAAAAAACAUUUAUUUUCAUUGCAAAAAAAAAAAAAAAA
AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
AAAAAAAA
SEQ ID NO: 19
> gE6 120A mRNA sequence 
GAGUUAGGAGUUGGUGGCAGUUUCAAUAACAGGUCAUUGCCGAGAAAAG
GAUAGCACUAUAAUAUGGGCACCGUGAACAAGCCCGUGGUGGGCGUGCU
GAUGGGCUUCGGCAUCAUCACCGGCACCCUGCGCAUCACCAACCCCGUGC
GGGCCAGCGUGCUGCGCUACGACGACUUCCACAUCGACGAGGACAAGCUG
GACACCAACUCCGUGUACGAGCCCUACUACCACAGCGACCACGCCGAGUC
 
CAGCUGGGUGAACCGGGGCGAGUCCAGCCGCAAGGCCUACGACCACAACU
CCCCCUACAUCUGGCCCCGGAACGACUACGACGGCUUCCUGGAGAACGCC
CACGAGCACCACGGGGUGUACAACCAGGGCCGCGGGAUCGACAGCGGCGA
GCGGCUGAUGCAGCCCACCCAGAUGUCCGCCCAGGAGGACCUGGGGGACG
ACACCGGCAUCCACGUGAUCCCCACCCUGAACGGGGACGACCGCCACAAG
AUCGUGAACGUGGACCAGCGGCAGUACGGCGACGUGUUCAAGGGGGACC
UGAACCCCAAGCCCCAGGGCCAGCGCCUGAUCGAGGUCAGCGUGGAGGAG
AACCACCCCUUCACCCUGCGGGCCCCCAUCCAGCGCAUCUACGGGGUCCG
GUACACCGAGACCUGGUCCUUCCUGCCCAGCCUGACCUGCACCGGCGACG
CCGCCCCCGCCAUCCAGCACAUCUGCCUGAAGCACACCACCUGCUUCCAG
GACGUGGUCGUGGACGUCGACUGCGCCGAGAACACCAAGGAGGACCAGCU
CGCCGAGAUCUCCUACCGCUUCCAGGGGAAGAAGGAGGCCGACCAGCCCU
GGAUCGUGGUCAACACCAGCACCCUGUUCGACGAGCUGGAGCUGGACCCG
CCCGAGAUCGAGCCCGGCGUGCUCAAGGUCCUGCGGACCGAGAAGCAGUA
CCUCGGGGUGUACAUCUGGAACAUGCGCGGCUCCGACGGGACCAGCACCU
ACGCCACCUUCCUGGUCACCUGGAAGGGCGACGAGAAGACCCGGAACCCC
ACCCCCGCCGUGACGCCCCAGCCGCGCGGGGCCGAGUUCCACAUGUGGAA
CUACCACUCCCACGUCUUCAGCGUGGGCGACACGUUCUCCCUCGCCAUGC
ACCUGCAGUACAAGAUCCACGAGGCCCCGUUCGACCUCCUGCUGGAGUGG
CUGUACGUCCCCAUCGACCCGACCUGCCAGCCCAUGCGGCUCUACAGCAC
GUGCCUGUACCACCCCAACGCCCCGCAGUGCCUCUCCCACAUGAACAGCG
GGUGCACCUUCACGUCCCCGCACCUGGCGCAGCGCGUGGCCAGCACCGUC
UACCAGAACUGCGAGCACGCGGACAACUACACCGCCUACUGCCUCGGCAU
CUCCCACAUGGAGCCCUCGUUCGGGCUGAUCCUCCACGACGGCGGGACGA
CCCUGAAGUUCGUGGACACGCCGGAGAGCCUCUCCGGCCUGUACGUCUUC
GUGGUCUACUUCAACGGGCACGUGGAGGCGGUCGCCUACACGGUGGUCUC
GACCGUGGACCACUUCGUCAACGCGAUCGAGGAGCGGGGCUUUCCCCCGA
CCGCCGGGCAGCCGCCCGCGACGACGAAGCCGAAGGAGAUCACCCCGGUG
AACCCCGGCACGAGCCCGCUCCUGCGCUACGCCGCGUGGACCGGGGGCCU
CGCCGCGGUCGUGCUGCUCUGCCUGGUCAUCUUCCUCAUCUGCACCGCCA
AGCGGAUGCGCGUGAAGGCGUACCGGGUCGACAAGUCCCCCUACAACCAG
UCGAUGUACUACGCCGGGCUGCCCGUGGACGACUUCGAGGACAGCGAGUC
CACGGACACCGAGGAGGAGUUCGGCAACGCGAUCGGGGGCUCGCACGGGG
GCUCCUCGUACACGGUCUACAUCGACAAGACCCGCUGAUGAACCGGUGCU
CGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUC
UGCCUAAUAAAAAACAUUUAUUUUCAUUGCAAAAAAAAAAAAAAAAAAAA
AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
AAAAA
SEQ ID NO: 20
> Amino acid sequence of gE1 
MGTVNKPVVGVLMGFGIITGTLRITNPVRASVLRYDDFHIDEDKLDTNSVYEPY
YHSDHAESSWVNRGESSRKAYDHNSPYIWPRNDYDGFLENAHEHHGVYNQGR
GIDSGERLMQPTQMSAQEDLGDDTGIHVIPTLNGDDRHKIVNVDQRQYGDVFK
GDLNPKPQGQRLIEVSVEENHPFTLRAPIQRIYGVRYTETWSFLPSLTCTGDAAP
AIQHICLKHTTCFQDVVVDVDCAENTKEDQLAEISYRFQGKKEADQPWIVVNT
STLFDELELDPPEIEPGVLKVLRTEKQYLGVYIWNMRGSDGTSTYATFLVTWKG
DEKTRNPTPAVTPQPRGAEFHMWNYHSHVFSVGDTFSLAMHLQYKIHEAPFDL
LLEWLYVPIDPTCQPMRLYSTCLYHPNAPQCLSHMNSGCTFTSPHLAQRVASTV
YQNCEHADNYTAYCLGISHMEPSFGLILHDGGTTLKFVDTPESLSGLYVFVVYF
NGHVEAVAYTVVSTVDHFVNAIEERGFPPTAGQPPATTKPKEITPVNPGTSPLLRY
AAWTGGLA
SEQ ID NO: 21
> Amino acid sequence of gE2 
MGTVNKPVVGVLMGFGIITGTLRITNPVRASVLRYDDFHIDEDKLDTNSVYEPY
YHSDHAESSWVNRGESSRKAYDHNSPYIWPRNDYDGFLENAHEHHGVYNQGR
GIDSGERLMQPTQMSAQEDLGDDTGIHVIPTLNGDDRHKIVNVDQRQYGDVFK
GDLNPKPQGQRLIEVSVEENHPFTLRAPIQRIYGVRYTETWSFLPSLTCTGDAAP
AIQHICLKHTTCFQDVVVDVDCAENTKEDQLAEISYRFQGKKEADQPWIVVNT
STLFDELELDPPEIEPGVLKVLRTEKQYLGVYIWNMRGSDGTSTYATFLVTWKG
DEKTRNPTPAVTPQPRGAEFHMWNYHSHVFSVGDTFSLAMHLQYKIHEAPFDL
LLEWLYVPIDPTCQPMRLYSTCLYHPNAPQCLSHMNSGCTFTSPHLAQRVASTV
YQNCEHADNYTAYCLGISHMEPSFGLILHDGGTTLKFVDTPESLSGLYVFVVYF
NGHVEAVAYTVVSTVDHFVNAIEERGFPPTAGQPPATTKPKEITPVNPGTSPLLRY
AAWTGGLAAVVLLCLVIFLICTAKRMRVKAARVDKS
SEQ ID NO: 22
> Amino acid sequence of gE3 
MGTVNKPVVGVLMGFGIITGTLRITNPVRASVLRYDDFHIDEDKLDTNSVYEPY
YHSDHAESSWVNRGESSRKAYDHNSPYIWPRNDYDGFLENAHEHHGVYNQGR
GIDSGERLMQPTQMSAQEDLGDDTGIHVIPTLNGDDRHKIVNVDQRQYGDVFK
GDLNPKPQGQRLIEVSVEENHPFTLRAPIQRIYGVRYTETWSFLPSLTCTGDAAP
AIQHICLKHTTCFQDVVVDVDCAENTKEDQLAEISYRFQGKKEADQPWIVVNT
STLFDELELDPPEIEPGVLKVLRTEKQYLGVYIWNMRGSDGTSTYATFLVTWKG
DEKTRNPTPAVTPQPRGAEFHMWNYHSHVFSVGDTFSLAMHLQYKIHEAPFDL
LLEWLYVPIDPTCQPMRLYSTCLYHPNAPQCLSHMNSGCTFTSPHLAQRVASTV
YQNCEHADNYTAYCLGISHMEPSFGLILHDGGTTLKFVDTPESLSGLYVFVVYF
NGHVEAVAYTVVSTVDHFVNAIEERGFPPTAGQPPATTKPKEITPVNPGTSPLLRY
AAWTGG
SEQ ID NO: 23
> Amino acid sequence of gE4 MGTVNKPVVGVLMGFGIITGTLRITNPVRASVLRYDDFHIDEDKLDTNSVYEPY 
YHSDHAESSWVNRGESSRKAYDHNSPYIWPRNDYDGFLENAHEHHGVYNQGR
GIDSGERLMQPTQMSAQEDLGDDTGIHVIPTLNGDDRHKIVNVDQRQYGDVFK
GDLNPKPQGQRLIEVSVEENHPFTLRAPIQRIYGVRYTETWSFLPSLTCTGDAAP
AIQHICLKHTTCFQDVVVDVDCAENTKEDQLAEISYRFQGKKEADQPWIVVNT
STLFDELELDPPEIEPGVLKVLRTEKQYLGVYIWNMRGSDGTSTYATFLVTWKG
DEKTRNPTPAVTPQPRGAEFHMWNYHSHVFSVGDTFSLAMHLQYKIHEAPFDL
LLEWLYVPIDPTCQPMRLYSTCLYHPNAPQCLSHMNSGCTFTSPHLAQRVASTV
YQNCEHADNYTAYCLGISHMEPSFGLILHDGGTTLKFVDTPESLSGLYVFVVYF
NGHVEAVAYTVVSTVDHFVNAIEERGFPPTAGQPPATTKPKEITPVNPGTSPLLRY
AAWTGGLAAVVLLCLVIFLICTAKRMRVKAYRVDKS
SEQ ID NO: 24
> Amino acid sequence of gE5 
MGTVNKPVVGVLMGFGIITGTLRITNPVRASVLRYDDFHIDEDKLDTNSVYEPY
YHSDHAESSWVNRGESSRKAYDHNSPYIWPRNDYDGFLENAHEHHGVYNQGR
GIDSGERLMQPTQMSAQEDLGDDTGIHVIPTLNGDDRHKIVNVDQRQYGDVFK
GDLNPKPQGQRLIEVSVEENHPFTLRAPIQRIYGVRYTETWSFLPSLTCTGDAAP
AIQHICLKHTTCFQDVVVDVDCAENTKEDQLAEISYRFQGKKEADQPWIVVNT
STLFDELELDPPEIEPGVLKVLRTEKQYLGVYIWNMRGSDGTSTYATFLVTWKG
DEKTRNPTPAVTPQPRGAEFHMWNYHSHVFSVGDTFSLAMHLQYKIHEAPFDL
LLEWLYVPIDPTCQPMRLYSTCLYHPNAPQCLSHMNSGCTFTSPHLAQRVASTV
YQNCEHADNYTAYCLGISHMEPSFGLILHDGGTTLKFVDTPESLSGLYVFVVYF
NGHVEAVAYTVVSTVDHFVNAIEERGFPPTAGQPPATTKPKEITPVNPGTSPLLRY
AAWTGGLAAVVLLCLVIFLICTA
SEQ ID NO: 25
> Luciferase mRNA sequence

UAAUACGACUCACUAUAAGGGAAGCUUCUCUCGCACUCUGUUCUUCCGCC
GCUCCGCCGUCGCGUUUCUCUGCCGGUCGCAGCUAGCCUUAAGGCCACCA
UGGAAGACGCCAAAAACAUAAAGAAAGGCCCGGCGCCAUUCUAUCCGCUG
GAAGAUGGAACCGCUGGAGAGCAACUGCAUAAGGCUAUGAAGAGAUACG
CCCUGGUUCCUGGAACAAUUGCUUUUACAGAUGCACAUAUCGAGGUGGA
CAUCACUUACGCUGAGUACUUCGAAAUGUCCGUUCGGUUGGCAGAAGCU
AUGAAACGAUAUGGGCUGAAUACAAAUCACAGAAUCGUCGUAUGCAGUG
AAAACUCUCUUCAAUUCUUUAUGCCGGUGUUGGGCGCGUUAUUUAUCGG
AGUUGCAGUUGCGCCCGCGAACGACAUUUAUAAUGAACGUGAAUUGCUC
AACAGUAUGGGCAUUUCGCAGCCUACCGUGGUGUUCGUUUCCAAAAAGG
GGUUGCAAAAAAUUUUGAACGUGCAAAAAAAGCUCCCAAUCAUCCAAAA
AAUUAUUAUCAUGGAUUCUAAAACGGAUUACCAGGGAUUUCAGUCGAUG
UACACGUUCGUCACAUCUCAUCUACCUCCCGGUUUUAAUGAAUACGAUUU
UGUGCCAGAGUCCUUCGAUAGGGACAAGACAAUUGCACUGAUCAUGAAC
UCCUCUGGAUCUACUGGUCUGCCUAAAGGUGUCGCUCUGCCUCAUAGAAC
UGCCUGCGUGAGAUUCUCGCAUGCCAGAGAUCCUAUUUUUGGCAAUCAA
AUCAUUCCGGAUACUGCGAUUUUAAGUGUUGUUCCAUUCCAUCACGGUU
UUGGAAUGUUUACUACACUCGGAUAUUUGAUAUGUGGAUUUCGAGUCGU
CUUAAUGUAUAGAUUUGAAGAAGAGCUGUUUCUGAGGAGCCUUCAGGAU
UACAAGAUUCAAAGUGCGCUGCUGGUGCCAACCCUAUUCUCCUUCUUCGC

CAAAAGCACUCUGAUUGACAAAUACGAUUUAUCUAAUUUACACGAAAUU
GCUUCUGGUGGCGCUCCCCUCUCUAAGGAAGUCGGGGAAGCGGUUGCCAA
GAGGUUCCAUCUGCCAGGUAUCAGGCAAGGAUAUGGGCUCACUGAGACU
ACAUCAGCUAUUCUGAUUACACCCGAGGGGGAUGAUAAACCGGGCGCGG
UCGGUAAAGUUGUUCCAUUUUUUGAAGCGAAGGUUGUGGAUCUGGAUAC
CGGGAAAACGCUGGGCGUUAAUCAAAGAGGCGAACUGUGUGUGAGAGGU
CCUAUGAUUAUGUCCGGUUAUGUAAACAAUCCGGAAGCGACCAACGCCUU
GAUUGACAAGGAUGGAUGGCUACAUUCUGGAGACAUAGCUUACUGGGAC
GAAGACGAACACUUCUUCAUCGUUGACCGCCUGAAGUCUCUGAUUAAGU
ACAAAGGCUAUCAGGUGGCUCCCGCUGAAUUGGAAUCCAUCUUGCUCCAA
CACCCCAACAUCUUCGACGCAGGUGUCGCAGGUCUUCCCGACGAUGACGC
CGGUGAACUUCCCGCCGCCGUUGUUGUUUUGGAGCACGGAAAGACGAUG
ACGGAAAAAGAGAUCGUGGAUUACGUCGCCAGUCAAGUAACAACCGCGA
AAAAGUUGCGCGGAGGAGUUGUGUUUGUGGACGAAGUACCGAAAGGUCU
UACCGGAAAACUCGACGCAAGAAAAAUCAGAGAGAUCCUCAUAAAGGCC
AAGAAGGGCGGAAAGAUCGCCGUGGAUUACAAGGAUGACGACGAUAAGU
AAGGGCCCUCUGCCGUGGGCCUGUCGUGCCAGUCCUGGGGGCGAGAUCGG
GAGAAUCACGUCCUGGAACUGCAUGUUCUUGCGACUCUUGGGACUUCAUC
UUAACUUCUCGCUGCCCCAGCCAUGUUUUCAACCAUGGCAUCCCUCCCCC
AAUUAGUUCCCUGUCAUCCUCGUCAACCUUCUCUGUAAGUGCCUGGUAAG
CUUGCCCUUGCUUAAGAACUCAAAACAUAGCUGUGCUCUACCGCGGAAAA
AAAAAAAAAAAAAAAAAAAAAAAAAAGCAUAUGACUAAAAAAAAAAAAA
AAAAAAAAAAAAAAAAAAAAAAAAAAAAAAAAAAAAAAAAAAAAAAAAAA
AAAAAAAA
SEQ ID NO: 26

## Claims

1. A pharmaceutical composition, comprising a vector, wherein the vector comprises a cationic lipid, and the cationic lipid comprises the compound represented by formula I or a pharmaceutically acceptable salt thereof; a molar percentage of the cationic lipid to the vector is greater than or equal to 10% and less than 50%:

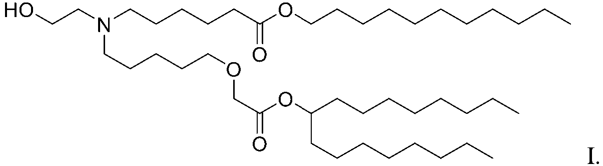

2. The pharmaceutical composition according to claim 1, wherein the vector further comprises a non-cationic lipid, and the non-cationic lipid is selected from the group consisting of at least one of a phospholipid and cholesterol or a derivative thereof.

3. The pharmaceutical composition according to claim 2, wherein the phospholipid is selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoyl phosphatidylglycerol (DPPG), palmitoyl oleoyl phosphatidylethanolamine (POPE), distearoyl-phosphatidyl-ethanolamine (DSPE), dipalmitoyl phosphatidylethanolamine (DPPE), dimyristoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and a mixture thereof, preferably from the group consisting of 1,2-distearoyl-sn-glycero-3-phosphocholine (DSPC) and 1,2-dioleoyl-sn-glycero-3-phosphoethanolamine (DOPE).

4. The pharmaceutical composition according to claim 2 or 3, wherein a content of the phospholipid accounts for 5-40%, preferably 10-20%, for example, 10%, of a molar amount of the vector.

5. The pharmaceutical composition according to any one of claims 2-4, wherein cholesterol or the derivative thereof accounts for 30-60%, preferably 35-45%, for example, 40.5%, of the molar amount of the vector.

6. The pharmaceutical composition according to any one of claims 1-5, wherein the vector further comprises a conjugated lipid; preferably, the conjugated lipid is PEG-modified phosphatidylethanolamine, PEG-modified phosphatidic acid, PEG-modified ceramide, PEG-modified dialkylamine, PEG-modified diacylglycerol, or PEG-modified dialkylglycerol; more preferably, the conjugated lipid is distearoylphosphatidylethanolamine polyethylene glycol 2000 (DSPE-PEG2000), dimyristoylglycero-3-methoxypolyethylene glycol 2000 (DMG-PEG2000), and methoxypolyethylene glycol ditetradecylacetamide (ALC-0159).

7. The pharmaceutical composition according to claim 6, wherein the conjugated lipid accounts for 0.5-4%, preferably 1.5-2.5%, for example, 1.5%, of the molar amount of the vector.

8. The pharmaceutical composition according to any one of claims 1-7, comprising an active agent, wherein the active agent is selected from the group consisting of a nucleic acid, preferably from the group consisting of a ribonucleic acid and a deoxyribonucleic acid, and more preferably from the group consisting of a small interfering RNA, a microRNA, a small hairpin RNA, a messenger RNA (mRNA), a circular RNA, a self-replicating RNA, and a mixture thereof.

9. The pharmaceutical composition according to claim 8, wherein the mRNA comprises an open reading frame (ORF), and the ORF encodes a protein or polypeptide;
preferably, the mRNA further comprises any one or any combination of a 5' untranslated region element (5'UTR), a 3' untranslated region element (3'UTR), and a polyadenylic acid (poly-A) tail;
preferably, the mRNA further comprises a 5'Cap structure.

10. The pharmaceutical composition according to claim 9, wherein the protein or polypeptide is an antigen;
preferably, the antigen is selected from the group consisting of a viral antigen, a bacterial antigen, and a cancer antigen;
more preferably, the viral strain is selected from the group consisting of an influenza virus, a coronavirus, varicella zoster virus (VZV), and respiratory syncytial virus (RSV).

11. The pharmaceutical composition according to claim 10, wherein the antigen is a varicella zoster virus (VZV) glycoprotein E (gE) protein whose amino acid sequence is set forth in any one of SEQ ID NO: 1 or SEQ ID NO: 21-SEQ ID NO: 25;
preferably, a nucleotide sequence of the antigen is set forth in any one of SEQ ID NO: 3-SEQ ID NO: 8;
more preferably, the nucleotide sequence of the mRNA is set forth in any one of SEQ ID NO: 13-SEQ ID NO: 20.

12. The pharmaceutical composition according to claim 9, wherein the protein or polypeptide is hepatocyte growth factor (HGF), an antibody, or an antigen-binding fragment thereof.

13. The pharmaceutical composition according to any one of claims 1-12, comprising:
a) an active agent, preferably an mRNA;
b) a cationic lipid comprising the compound represented by formula I or a pharmaceutically acceptable salt thereof, wherein a molar percentage of the cationic lipid to the vector is greater than or equal to 10% and less than 50%: 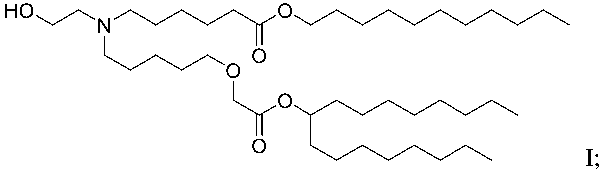
c) a non-cationic lipid selected from the group consisting of a phospholipid and cholesterol or a derivative thereof, wherein the phospholipid accounts for 5-40% of the molar amount of the vector, and cholesterol or the derivative thereof accounts for 30-60% of the molar amount of the vector;
and d) a conjugated lipid, wherein a molar percentage content of the conjugate in the vector is 0.5-4%.

14. The pharmaceutical composition according to any one of claims 1-13, wherein the pharmaceutical composition is a nanoparticle formulation, and the nanoparticle formulation has an average particle size of 10 nm-220 nm.

15. A freeze-dried formulation, wherein the freeze-dried formulation is obtained by freeze-drying the pharmaceutical composition according to any one of claims 1-14.

16. A reconstituted solution, wherein the reconstituted solution is obtained by a step of reconstituting the freeze-dried formulation according to claim 15, and a preferred solvent for the reconstitution is water for injection.

17. A method for preparing the pharmaceutical composition according to any one of claims 1-14, comprising a step of mixing the cationic lipid, the non-cationic lipid, and the conjugated lipid in an organic solvent.

18. Use of the pharmaceutical composition according to any one of claims 1-14, the freeze-dried formulation according to claim 15, or the reconstituted solution according to claim 16 in the manufacture of a medicament for inducing a protective immune response in a mammal, preferably in the manufacture of a vaccine.

19. Use of the pharmaceutical composition according to any one of claims 1-14, the freeze-dried formulation according to claim 15, or the reconstituted solution according to claim 16 in the manufacture of a medicament for treating a disease or dysfunction in a mammal, wherein the disease is preferably a viral infection or cancer.
